# EUROPEAN PATENT SPECIFICATION

(11) **EP 3 844 309 B1**
(45) Date of publication and mention of the grant of the patent: **19.07.2023**
(21) Application number: 19779538.8
(22) Date of filing: 29.08.2019
(51) Int. Cl.: C12Q 1/6886

(54) **A METHOD FOR DIAGNOSING CANCERS OF THE GENITOURINARY TRACT**
EINE METHODE ZUR DIAGNOSE VON KREBSERKRANKUNGEN DES UROGENITALTRAKTS
MÉTHODE DE DIAGNOSTIC DES CANCERS DU TRACTUS GÉNITO-URINAIRE

(30) Priority: 29.08.2018 GB 201814040
(43) Date of publication of application: 07.07.2021
(73) Proprietor: Oncologica UK Ltd, Cambridge, Cambridgeshire CB10 1XL (GB)
(72) Inventor: LODDO, Marco, Saffron Walden CB11 4AF (GB); WILLIAMS, Gareth, Waterside Ely CB7 4AU (GB); HARDISTY, Keeda-Marie, Saffron Walden CB10 2BD (GB)
(74) Representative: London IP Ltd
(86) International application number: PCT/GB2019/052410
(87) International publication number: WO 2020/044046

(56) References cited:
- WO-A1-2015/041788
- WO-A1-2018/027178
- WO-A2-01/86288
- US-A1- 2014 303 001
- US-A1- 2017 145 518
- DATABASE EMBASE [Online] ELSEVIER SCIENCE PUBLISHERS, AMSTERDAM, NL; 1 July 2018 (2018-07-01), WANG A ET AL: "Urine - and blood-based DNA+RNA liquid biopsy tests in prostate, bladder and kidney cancers", XP002788473, Database accession no. EMB-623505426 & WANG A ET AL: "Urine - and blood-based DNA+RNA liquid biopsy tests in prostate, bladder and kidney cancers", CANCER RESEARCH 20180701 AMERICAN ASSOCIATION FOR CANCER RESEARCH INC. NLD, vol. 78, no. 13, Supplement 1, 1 July 2018 (2018-07-01), ISSN: 1538-7445
- FANG ZHAO ET AL: "Urinary DNA Methylation Biomarkers for Noninvasive Prediction of Aggressive Disease in Patients with Prostate Cancer on Active Surveillance", JOURNAL OF UROLOGY., vol. 197, no. 2, 1 February 2017 (2017-02-01), pages 335-341, XP055549741, BALTIMORE, MD, US ISSN: 0022-5347, DOI: 10.1016/j.juro.2016.08.081
- SUE-ING QUEK ET AL: "A Multiplex Assay to Measure RNA Transcripts of Prostate Cancer in Urine", PLOS ONE, vol. 7, no. 9, 20 September 2012 (2012-09-20), page e45656, XP055549751, DOI: 10.1371/journal.pone.0045656
- XIAO-YONG PU ET AL: "The value of combined use of survivin, cytokeratin 20 and mucin 7 mRNA for bladder cancer detection in voided urine", JOURNAL OF CANCER RESEARCH AND CLINICAL ONCOLOGY., vol. 134, no. 6, 20 November 2007 (2007-11-20), pages 659-665, XP055549753, DE ISSN: 0171-5216, DOI: 10.1007/s00432-007-0331-9
- VIRGINIA URQUIDI ET AL: "Urinary mRNA biomarker panel for the detection of urothelial carcinoma", ONCOTARGET, vol. 7, no. 25, 21 June 2016 (2016-06-21), XP055549756, DOI: 10.18632/oncotarget.9587
- MAFALDA PINTO ET AL: "Overexpression of the Mitotic Checkpoint Genes BUB1 and BUBR1 is Associated with Genomic Complexity in Clear Cell Kidney Carcinomas", CELLULAR ONCOLOGY, vol. 30, no. 5, 1 January 2008 (2008-01-01), pages 389-395, XP055549766, GB ISSN: 1570-5870, DOI: 10.3233/CLO-2008-0439
- IRIS LODEWIJK ET AL: "Liquid Biopsy Biomarkers in Bladder Cancer: A Current Need for Patient Diagnosis and Monitoring", INT. J. MOL. SCI., vol. 19, no. 9, 24 August 2018 (2018-08-24), page 2514, XP055634022, ISSN: 1661-6596, DOI: 10.3390/ijms19092514 -& K. M. PATEL ET AL: "Association Of Plasma And Urinary Mutant DNA With Clinical Outcomes In Muscle Invasive Bladder Cancer", SCIENTIFIC REPORTS, vol. 7, no. 1, 17 July 2017 (2017-07-17), XP055634027, DOI: 10.1038/s41598-017-05623-3

## Description

The present invention relates to a method for detecting or monitoring renal, bladder and/or prostate cancers. It further comprises the development of treatment regimes for selected patients, based upon the determination, and computers and devices programmed to carry out the determination.

### Background to the Invention

Genitourinary tract cancers including prostate, bladder and kidney cancers represent a major cause of morbidity and mortality worldwide. Bladder cancer is the 5th most common cancer in Europe with over 123,000 new cases every year resulting in 40,000 deaths. In Europe prostate cancer is ranked first among the most frequently diagnosed cancer among men, with around 345,000 new cases a year. There are around 88 400 new cases of kidney cancer per year making it the 10th most common cancer.

Haematuria is one of the most common findings on urinalysis in patients encountered by general practitioners with an incidence of 4 per 1000 patients per year; and it represents about 6% of new patients seen by urologists. Haematuria or irritative voiding are both symptoms of early transitional cell carcinoma. However, fewer than 1 in 10 people with haematuria actually have a genitourinary tract cancer. Haematuria or irritative voiding is more often related to less serious diseases such as urinary tract infections or benign prostatic hyperplasia. However, patients with these nonspecific symptoms may undergo extensive urological investigations, even though only a small percentage of them actually have malignancies. In this connection, the workup and screening of haematuria patients often requires cystoscopy. Cystoscopy is the gold standard diagnostic test for bladder cancer because it allows direct visualization and biopsy of the bladder urothelium.

As cystoscopy is invasive and also costly, both patients and clinicians would benefit greatly from the development of a cost-effective non-invasive test platform for the detection of genitourinary tract cancers. Moreover the diagnosis of genitourinary tract cancers at an early stage greatly improves outcomes following treatment interventions such as surgery, radiotherapy, chemotherapy and molecular targeted therapies/immunotherapies.

To date, urine tests for detecting bladder cancer have focused on the detection of one or more protein biomarkers using immunoassay techniques (see for example Kelly et al. PLoS ONE 7(7): e40305). A particular biomarker detected is the MCM5.

Mcm2-7 protein expression in normal epithelium is restricted to the basal stem/transit amplifying compartments and is absent from surface layers as cells adopt a fully differentiated phenotype. Superficial cells obtained either through exfoliation or by surface sampling should therefore be negative for Mcm2-7 proteins. In premalignant (dysplastic) epithelial lesions and in malignancy there is an expansion of the proliferative compartment coupled to arrested differentiation, resulting in the appearance of proliferating, MCM-positive cells in superficial layers. Immunodetection of Mcm2-7 protein in exfoliated or surface-sampled cells is thus indicative of an underlying premalignant/dysplastic lesion or malignancy.

One of these cell cycle regulated proteins is Mcm5 which represents a component of the DNA helicase and, therefore, is a potential biomarker for cancer detection. It has been shown by immunocytochemical methods for detection of Mcm5 or alternatively application of a 12A7-4B4 sandwich Mcm5 immunoassay that this approach can be used for the detection of a wide range of cancers including cervical, oesophageal, prostate, and bladder, renal and pancreatic cancers including cholangiocarcinoma.

However, the detection of one or more protein biomarkers of the this type using immunoassay can result in erroneous results, and in particular, false positives.

Urine tests can be used in such immunoassays as disruption of the normal process of differentiation during the formation of cancer results in tumour cells being locked into the proliferative cell cycle. This results in the elevation of a range of cell cycle regulated proteins in the tumour cells shed into body fluids such as urine.

Wang *et al,* American Association for Cancer Research vol 78, no 13, Supplement 1, 1 July 2018 discloses a next-generation sequencing based multiplex test for prostate, bladder and kidney cancer along with genomic profiling of both cfDNA and cfRNA in blood and urine samples of patients with such cancers.

### Summary of the Invention

According to the present invention there is provided a method for detecting or monitoring renal, bladder and/or prostate cancer, said method comprising analysing nucleic acid obtained from a urine sample from a subject, detecting the presence of an aberration selected from a single nucleotide variant, indel, deletion, amplication and/or fusion in a coding sequence , or increased or decreased gene expression from a coding sequence, of at least one biomarker from each of Tables 1 to 7, as well as, the tumour mutational burden (TMB), and relating the presence of said biomarkers to the presence of malignancy wherein an algorithm indicative of the presence or level of malignancy is applied to the results obtained wherein a score of '0' is applied to results which show no changes over wild type or normal expression profiles of the various biomarkers;
a score of '1' is applied in the case of the presence of an oncogenic mutation in a biomarker gene, or to the presence of 2 to 3 additional copies of a biomarker gene, or for the presence of an oncogenic gene fusion, or up to a 500 nRPM change in RNA expression of a biomarker gene, or for a TMB of <10mutations/Megabase;
a score of '2' is applied in the case of the presence of from 4 to 8 additional copies of a biomarker gene; or to 500 to 1500 nRPM change in RNA expression of a biomarker gene;
a score of '3' is applied in the case of the presence of from more than 8 additional copies of a biomarker gene; or to a >1500 nRPM change in RNA expression of a biomarker gene, or for a TMB of >10 mutations/Megabase;
wherein an overall score of 0 is indicative of no malignancy, a score of 1 to 2 is indicative of a malignancy with intermediate specificity and high sensitivity, a score of 3 to 5 is indicative of a malignancy with high specificity and high sensitivity, and a score in excess of 6 is indicative of malignancy with very high specificity and very high sensitivity.

Preferably the sample is a formalin fixed sample.

Preferably as a preliminary step, extracting nucleic acid from the sample.

Preferably the results obtained are further used to identify susceptibility of a subject to PD-1 or PD-L1 targeted therapy or treatment.

Preferably at least 10 of the biomarkers in Tables 1 to 7 are detected.

Preferably all the biomarkers listed in Tables 1 to 7 are detected.

Preferably the subject is an apparently healthy individual.

Preferably the subject has been previously diagnosed with renal, bladder and/or prostate cancer and is undergoing treatment or therapy therefor, and wherein the method is carried out repeatedly over time, to monitor the efficacy of the treatment or therapy.

Preferably the results are used to decide to modify or expand on the said treatment or therapy.

Preferably the subject is suffering from haematuria.

Preferably the method is carried out using a high-throughput assay platform.

Preferably the results are analysed to produce a customised recommendation for treatment of the cancer.

According to the present invention there is provided a system for for detecting or monitoring renal, bladder and/or prostate cancer, said system comprising:
a processor; and
a memory that stores code of an algorithm that, when executed by the processor, causes the computer system to:
   receive input levels of the biomarkers of the method;
   receive a further input level relating to the tumour mutational burden of nucleic acid in the urine sample;
   analyze and transform the input levels via an algorithm to provide an output indicating the presence or level of malignancy present;
   display the output on a graphical interface of the processor
   wherein the algorithm applied is:
      a score of '0' is applied to results which show no changes over wild type or normal expression profiles of the various biomarkers;
      a score of '1' is applied in the case of the presence of an oncogenic mutation in a biomarker gene, or to the presence of 2 to 3 additional copies of a biomarker gene, or for the presence of an oncogenic gene fusion, or up to a 500 nRPM change in RNA expression of a biomarker gene, or for a TMB of <10mutations/Megabase;
      a score of '2' is applied in the case of the presence of from 4 to 8 additional copies of a biomarker gene; or to 500 to 1500 nRPM change in RNA expression of a biomarker gene;
      a score of '3' is applied in the case of the presence of from more than 8 additional copies of a biomarker gene; or >1500 nRPM change in RNA expression of a biomarker gene, or for a TMB of >10 mutations/Megabase;
      wherein an overall score of 0 is indicative of no malignancy, a score of 1 to 2 is indicative of a malignancy with intermediate specificity and high sensitivity, a score of 3 to 5 is indicative of a malignancy with high specificity and high sensitivity, and a score in excess of 6 is indicative of malignancy with very high specificity and very high sensitivity.

Preferably the memory further comprises code to provide a customized recommendation for the treatment of the subject, based upon the input levels.

Preferably the customized recommendation is displayed on a graphical interface of the processor. According to the present invention there is provided a non-transitory computer-readable medium storing instructions that, when executed by a processor, cause a computer system to detect or monitor the level of renal, bladder nd/or prostate cancer in a urine sample of a subject, by:
receiving input levels of the biomarkers of the method and
receiving a further input level relating to the tumour mutational burden of nucleic acid in the urine sample;
analyzing and transforming the input levels via an algorithm to provide an output indicating the presence or level of malignancy present;
displaying the output on a graphical interface of the processor
wherein the algorithm applied is:
   a score of '0' is applied to results which show no changes over wild type or normal expression profiles of the various biomarkers;
   a score of '1' is applied in the case of the presence of an oncogenic mutation in a biomarker gene, or to the presence of 2 to 3 additional copies of a biomarker gene, or for the presence of an oncogenic gene fusion, or up to a 500 nRPM change in RNA expression of a biomarker gene, or for a TMB of <10mutations/Megabase;
   a score of '2' is applied in the case of the presence of from 4 to 8 additional copies of a biomarker gene; or to 500 to 1500 nRPM change in RNA expression of a biomarker gene;
   a score of '3' is applied in the case of the presence of from more than 8 additional copies of a biomarker gene; or >1500 nRPM change in RNA expression of a biomarker gene, or for a TMB of >10 mutations/Megabase;
   wherein an overall score of 0 is indicative of no malignancy, a score of 1 to 2 is indicative of a malignancy with intermediate specificity and high sensitivity, a score of 3 to 5 is indicative of a malignancy with high specificity and high sensitivity, and a score in excess of 6 is indicative of malignancy with very high specificity and very high sensitivity.

Preferably the non-transitory computer-readable medium further comprises storing instructions for developing a customized recommendation for treatment of the subject based upon the input levels and displaying the customized recommendation on a graphical interface of the processor.

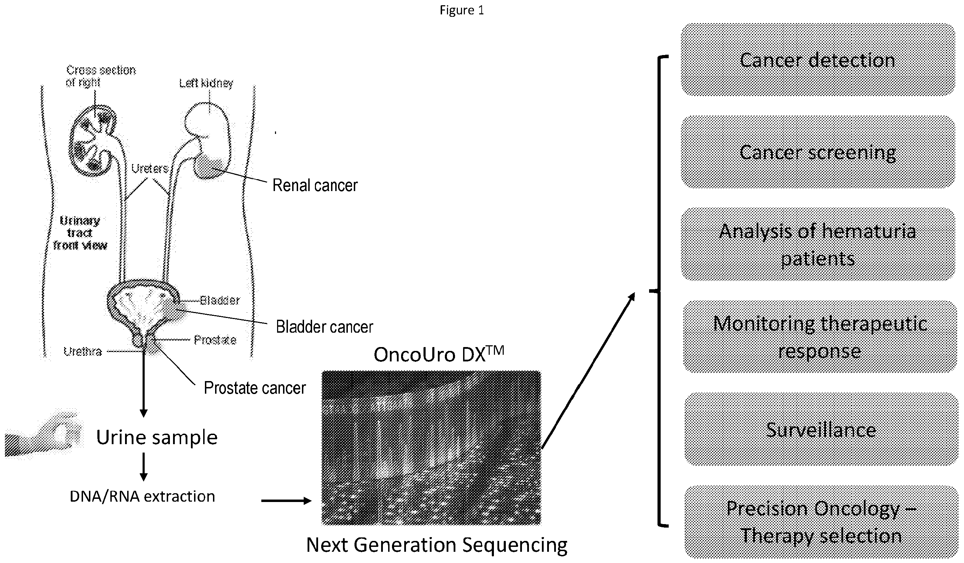

An illustration of the method is shown in Figure 1.

Levels of any specific RNA which would be considered to be elevated as compared to normal and therefore indicative of a positive biomarker are shown in Table 5, where expression cut-off values are listed.

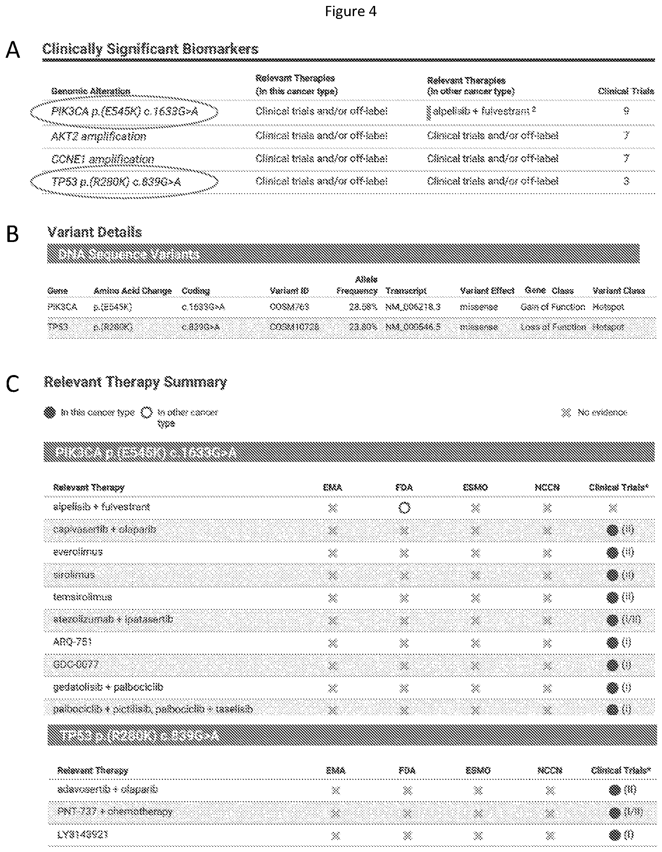

DNA from said urine sample is analysed and a mutation in a gene encoding a biomarker is detected that impacts on expression or function of the gene or gene product. Particular examples where actionable mutation, for example via an SNV hotspot mutation occurs, are found biomarkers listed in Tables 2 and 7 below. An example of SNV detection is shown in Figure 4. In particular, such biomarkers may be found in genes selected from the group consisting of ALK (anaplastic lymphoma kinase gene), BRAF (B-Raf gene), CD274 (PD-L1 gene), EGFR (epidermal growth factor receptor gene), ERBB2 (Receptor tyrosine-protein kinase erbB-2 or human epidermal growth factor receptor 2 gene), FGFR (fibroblast growth factor receptor gene), KIT (KIT proto-oncogene receptor tyrosine kinase gene), KRAS (K-ras gtpase gene), MET (MET proto-oncogene, receptor tyrosine kinase) or NRAS (N-ras protooncogene, GTPase gene). Specific mutations of these genes which are indicative of an oncogenic mutation are set out in Tables 2 and 7 below. These mutations may be detected using conventional techniques, as illustrated hereinafter.

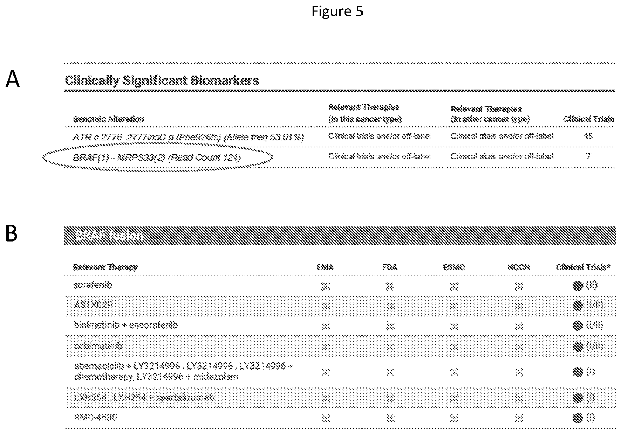

Biomarkers resulting from gene rearrangement leading to aberrant gene fusions may be detected, and these are listed in Tables 3 and 5 below. An example of detection of a gene fusion is shown in Figure 5. For example, it is recognised that the ALK gene may be fused with portions of the echinoderm microtubule-associated protein-like 4 (EML4) gene in some cancers, that FGFR genes may form fusions for instance with kinases in others, and that MET gene may become fused with other genes including for example TFG, CLIP2 or PTRZ1. Detection of any such fusion genes/proteins will provide a positive biomarker indication.

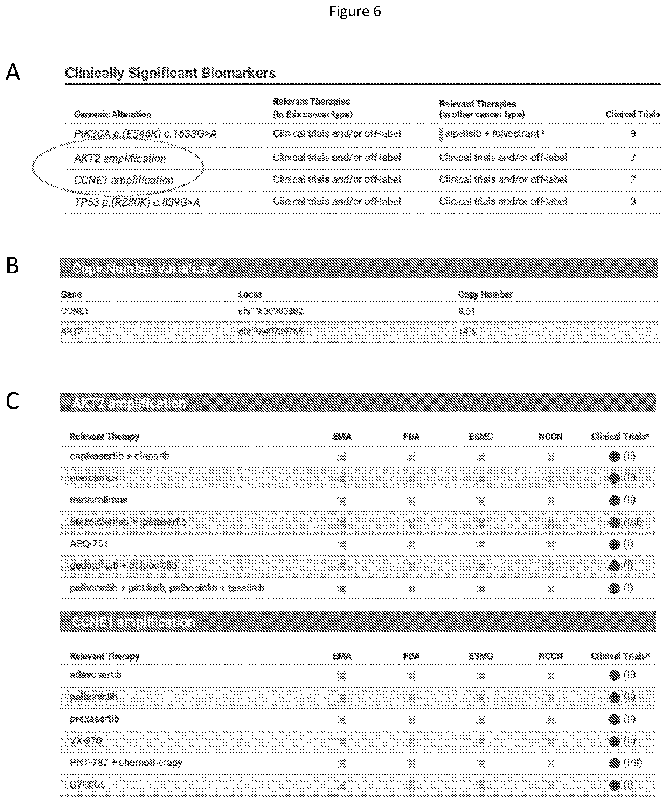

The analysis may identify the presence of copy number variants which may lead to increased expression. DNA from said sample can be analysed and the presence of a variation in copy number of a gene encoding a biomarker can be detected. Suitable biomarkers in this case are listed in Tables 4 and 6 below and include for example, ERBB2, FGFR, FGFR1, FCFR2, FGFR3, FGFR4, CD273 (PD-L2 gene) or CD273. In such cases, for example, an increase in copy number may result in amplification or increased expression and is indicative of malignancy. An example of CNV detection is shown in Figure 6. In such cases, the greater the increase in copy number the higher the susceptibility to therapies which target such genes may be indicated.

Unlike protein-based biomarkers, TMB is a quantitative measure of the total number of mutations per coding area of a tumor genome.

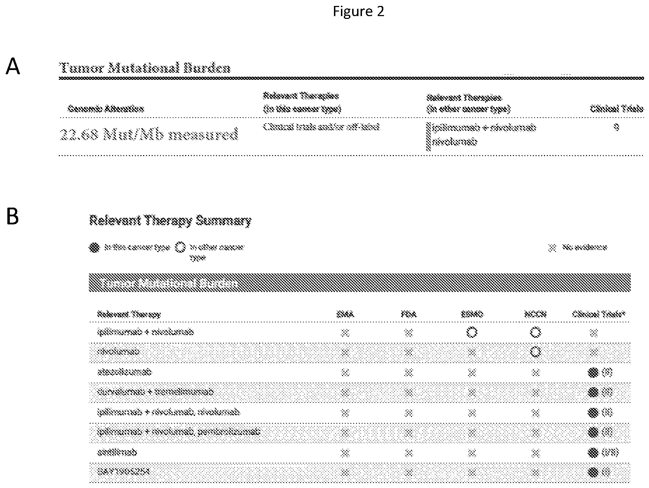

Since only a fraction of somatic mutations gives rise to neo-antigens, measuring the total number of somatic mutations (or TMB) within a particular coding area acts as a proxy for neo-antigen burden. The TMB may be measured using exome sequencing using Next Generation Sequence equipment in particular of 409 genes covering a 1.7 Mb coding region (Table 8). From all variants detected including non-synonomouse somatic mutations (SNVs and indels), all likely germline polymorphisms and predicted oncogenic drivers are removed from the analysis. The latter is performed to prevent ascertainment bias of sequencing known cancer genes. Tumour Mutational Burden is then calculated as mutations per Mb of DNA sequenced (mut/Mb). Analysis of TMB is both quantitative and qualitative and is reported as a metric (mut/Mb) as well as status. Status of High is classified as >10 mut/Mb, and Low <10 mut/Mb. An example of measurement of TMB is shown in Figure 2.

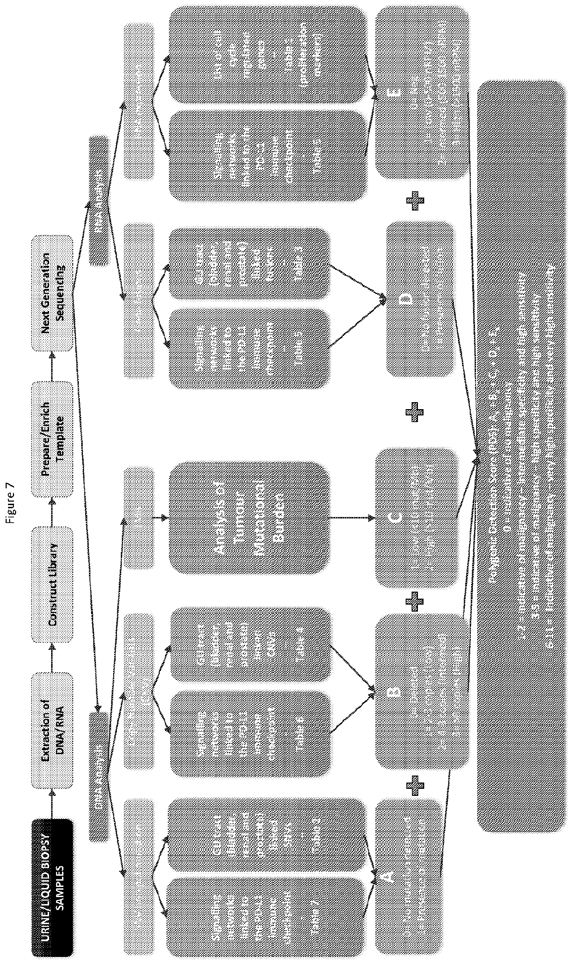

It has been recognised that TMB is a predictor of response to, for example, anti-PD-1/PD-L1/PD-L2 checkpoint inhibitors, and therefore it will provide a useful possible biomarker in the method of the invention.A particular example of the algorithm of the present invention is shown in Figure 7. In that case, a score 0 is indicative of no malignancy being present, a score of 1-2 is indicative of a malignancy, with intermediate specificity and high sensitivity, a score of 3-5 is indicative of a malignancy, with high specificity and high sensitivity and a score in excess of 6 is indicative of a malignancy, with very high specificity and very high sensitivity. Scores for A to E may not be present depending upon the analysis which has been undertaken. For example, analysis of SNV + CNV + RNA expression may take place so A + B +E would lead to the polygene detection score.

The method of the present invention can be performed by extracting DNA/RNA from, for example, a urine sample. The DNA/RNA samples are then run through Next Generation Sequencing equipment, for example, a ThermoFisher Ion Torrent using the method of the present invention which is known as OncoUro DX ^{™}. The various analytical methods and techniques for the determination of the biomarkers are suitably carried out in a high throughput assay platform as far as possible. The results thereof can be used in renal, bladder and/or prostate cancer detection, renal, bladder and/or prostate cancer screening, analysis of haematuria, monitoring therapeutic responses, surveillance, precision oncology therapy selection.

Analysis of a urine sample is non-invasive and the sample can be obtained by the patient themselves.

Nucleic acid, specifically RNA and DNA are extracted from the sample, and conveniently, this extraction forms a preliminary step in the method of the invention. The sample may be a fresh sample, but the method may also be carried out using stored samples, for example, on formalin fixed samples, or samples which have been preserved in either 10% neutral buffered formalin, formal saline solution, or alcohol and formalin based buffered solutions, e.g. Cytolyt^{®}, preservCyt or ColliPee. This means that samples may be prepared in one environment, such as a clinic or hospital, and then readily transferred to an appropriate laboratory for analysis in accordance with the method of the present invention.

As used herein, the term "biomarker" refers to any molecule, gene, sequence mutation or characteristic such as increased or decreased gene expression from a coding sequence of proliferative-linked genes. The biomarkers used in the present invention are identified in Tables 1 to 7. These may include mutations in the gene sequence, in particular 'hotspot' mutations which are known to give rise to oncological outcomes, copy number variations of genes, abherrant gene fusions or increased or decreased RNA expression as well as the tumour mutational burden.

By determining a range, in particular a wide range of biomarkers, associated with a wide spectrum of oncogenes, driver gene mutations, genomic instability as measured by tumour mutational burden, proliferation state and immune checkpoint regulation, a reliable diagnosis or monitoring of malignancy, such as a cancer, can be made with a high degree of sensitiviy and also specificity (i.e with a low frequency of false positives).

Biomarkers detected in the method of the invention are selected from each of Tables 1 to 7 and may be involved in cell cycle regulated genes, actionable genetic variants, biomarkers associated with signalling networks and cell cycle checkpoints.

Particular examples of cell cycle regulated genes are proliferation markers and particular examples of these are set out in Table 1. The detection of increased levels of such markers may be indicative of the presence of renal, bladder and/or prostate cancers. In general, these biomarkers are detected by analysis of RNA found in the sample, as would be understood in the art.

Actionable genetic variants which may be used as biomarkers in accordance with the invention include hotspot mutations such as those set out in Tables 2 and 7 hereinafter, aberrant gene fusions such as those set out in Tables 3 and 5 hereinafter, and copy number variants, such as those set out in Tables 4 and 6 hereinafter.

Preferably, at least 8 biomarkers in Tables 1 to 7 will be determined. Conveniently, at least 10, 20, 30 or 40 of the biomarkers in Tables 1 to 7 will be determined. Preferably all of the biomarkers in Tables 1 to 7. The larger the number of biomarkers used, the greater the probability that dysregulated genes will be identified, so that false negatives are avoided.

At least some of the biomarkers detected are associated with signalling networks, in particular those associated with the PD-L1 immune checkpoint. Such biomarkers are listed in Tables 5, 6 and 7 below. By analysing these biomarkers in particular, the susceptibility of a particular patient to anti-PD-1/PD-L1 directed therapy or immunotherapy can be assessed.

Anti-PD-1/PD-L1 directed immunotherapies have become one of the most important group of agents used in immunotherapy. The PD-1/PD-L1 pathway is normally involved in promoting tolerance and preventing tissue damage in the setting of chronic inflammation. Programmed death 1 (PD-1) and its ligands, PD-L1 and PD-L2, deliver inhibitory signals that regulate the balance between T cell activation, tolerance, and immunopathology. The PD-L1 is a transmembrane protein that binds to the PD-1 receptor during immune system modulation. This PD-1/PD-L1 interaction protects normal cells from immune recognition by inhibiting the action of T-cells thereby preventing immune-mediated tissue damage.

Harnessing the immune system in the fight against cancer has become a major topic of interest. Immunotherapy for the treatment of cancer is a rapidly evolving field from therapies that globally and non-specifically stimulate the immune system to more targeted approaches. The PD-1/PD-L1 pathway has emerged as a powerful target for immunotherapy. A range of cancer types have been shown to express PD-L1 which binds to PD-1 expressed by immune cells resulting in immunosupressive effects that allows these cancers to evade tumour destruction. The PD-1/PD-L1 interaction inhibits T-cell activation and augments the proliferation of T-regulatory cells (T-regs) which further suppresses the effector immune response against the tumour. This mimicks the approach used by normal cells to avoid immune recognition. Targeting PD-1/PD-L1 has therefore emerged as a new and powerful approach for immunotherapy directed therapies.

Targeting the PD-1/PD-L1 pathway with therapeutic antibodies directed at PD-1 and PD-L1 has emerged as a powerful therapy in those cancer types displaying features of immune evasion. Disrupting the PD-1/PD-L1 pathway with therapeutic antibodies directed against either PD-1 or PD-L1 (anti-PD-L1 or anti-PD-1 agents) results in restoration of effector immune responses with preferential activation of T-cells directed against the tumour.

There are many drugs in development targeting the PD-1/PD-L1 pathway including Pembrolizumab, atezolizumab, avelumab, nivolumab, durvalumab, PDR-001, BGB-A317, REG W2810, SHR-1210
Treatment with such agents or using a suitable immunotherapy approach may be indicated for patients determined to be susceptible to this type of therapy as a result of the presence of one or more of the biomarkers listed in Tables 5, 6 and 7 hereinafter.

At least some of the biomarkers detected are directly associated with the PD-1/PD-L1 pathway, and so is a biomarker of a gene selected from CD279 (PD1), CD274 (PD1)or CD273 (PD2). In a particular embodiment, both PD-L1 and PD-1 are assessed together providing a much more powerful assessment of the PD-1/PD-L1 signalling axis.

The method can be used to measure both PD-L1 and PD-L2 gene amplification (copy number variant; CNV) which has been linked to mRNA overexpression and may represent a much more reliable parameter to predict response to PD-1/PD-L1 inhibitors.

A range of biomarkers associated with different functions can be selected. In this connection, it has been identified that choosing a range of biomarkers provides a better indication of susceptibility to treatment which targets an immune pathway and in particular, the PD-1/PD-L1. Mutations in other genes, in particular oncogenic mutations, are likely to give rise to so-called 'neo-antigens'. Neo-antigens are newly formed antigens that have not previously been recognised by the immune system. When the neo-antigens are cancer-specific antigens, this can result in T-cell activation against cancer cells if the immune system is effective and not subject to suppression. Therefore, where neo-antigens are present, patients may show a more efficient and durable response to agents which act on immune pathways such as the PD-1/PD-L1 pathway.

The method of the invention may be utilised in screening methods for renal, bladder and/or prostate cancer. Accordingly, the subjects may be apparently healthy individuals, and the screening may take place on large numbers of subjects. As a urine sample is used, the non-invasive nature of the method of the invention makes it particularly suitable for this type of large-scale screening process. This may be advantageous in allowing the early stage detection of renal, bladder and/or prostate cancers in subjects who have not demonstrated any symptoms.

However, the method of the invention may also be utilised to monitor the progress of a malignancy in a subject who has been previously diagnosed with renal, bladder and/or prostate cancer. In such cases, the method may be carried out repeatedly over a period of time, for example, over a period of weeks, months or even years, whilst a particular treatment or therapy, such as chemotherapy, immunotherapy or radiotherapy, is being administered, and the results used to monitor the efficacy of a particular treatment or therapy on the progress of the disease. In particular, the method of the invention can be used to measure a therapeutic response, for example by detecting a decrease in particular biomarkers such as cell cycle regulated proteins, or a decrease in mutations associated with oncogenes or tumours.

The results may then be used to direct the continued treatment, for example by modifying or expanding on the existing treatment. Preferably, the method of the invention, by identifying specific biomarkers, which include actionable mutations and immune checkpoint regulators, allows for the development of targeted therapies and immunotherapies that are specific for a subject's particular cancer, such as anti PD-1 or PD-L1 therapies as discussed above.

Conveniently, the subject is suffering from haematuria. Using the method of the invention, it would be possible to identify the very low percentage of haematuria patients who harbour a renal, bladder and/or prostate cancer. This will circumvent the costly and invasive procedure of cystoscopy for patients with benign haematuria.

The biomarkers in Tables 1 to 7 were identified using DNA or RNA analysis or a combination thereof. Nucleic acids (DNA and/or RNA) extracted from a sample as described above is used to construct a library, using conventional methods, for example as outlined below. The library is enriched as necessary and then used as a template for enrichment, again using conventional methods as outlined below. Analysis of this is then carried out using semiconductor Next Generation Sequencing equipment such as sold by ThermoFisher under the trade mark Ion Torrent using the method set out in the present invention.

Preferably the method of the invention uses targeted semiconductor sequencing to cover the entire coding regions of the biomarkers of interest. Amplicons can be designed to overlap for sequence coverage redundancy and to be able to amplify fragmented DNA templates obtained fromsamples. The method is therefore able to identify a wide variety of actionable genetic variants including point mutations, deletions, duplications, and insertions.

Thus, conveniently, the method of the invention utilises Next Generation Sequencing technology to quantitate biomarkers present in a urine sample. In this way, the method of the invention can be carried out using only a small amount of sample, such as may be found in a urine sample, and it may be optimised for analysis of degraded DNA/RNA. Further, a quantitative test gives a much more accurate method than immunohistochemistry
Biomarkers identified in Table 1 and 5 below.may be most readily identified by an analysis of gene expression, for example, using quantitative measurement of RNA transcripts (i.e thelevels of RNA are analysed, wherein a change in the expression level as compared to wild type is indicative of malignancy. Conveniently, in tumours where the PD-L1 checkpoint is implicated, elevated levels of RNA from CD273 (PD-L2), CD274 (PD-L1) and CD279 (PD-1) may be detected.

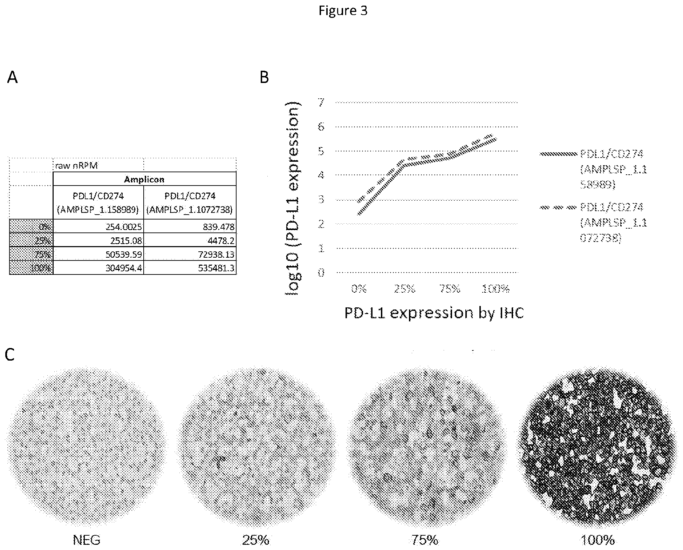

Preferably, gene expression at multiple exon-intron loci across mRNAs such as PD-L1 and PD-1 mRNAs is carried out, which is then coupled to a bioinformatics programme that normalises gene expression across the whole gene allowing very accurate quantitative measurement of RNA expression levels. The analytical validation of PD-L1 mRNA expression is shown in Figure 3.

In addition, elevated levels of RNA of NFATC1 (Nuclear Factor Of Activated T-Cells 1), PIK3CA (Phosphatidylinositol-4,5-Bisphosphate 3-Kinase Catalytic Subunit Alpha), PIK3CD (Phosphatidylinositol-4,5-Bisphosphate 3-Kinase Catalytic Subunit Delta), PRDM1 (PR domain zinc finger protein 1), PTEN, (Phosphatase and tensin homolog), PTPN11 (Tyrosine-protein phosphatase non-receptor type 11), MTOR (mechanistic target of rapamycin), HIF1a (Hypoxia-inducible factor 1-alpha) and FOXOlm (forkhead box class 01 mutant) may be quantified. In addition, the method of the invention may detect loss of gene expression of the mismatch repair genes MLH1, PMS2, MSH6 and MLH2. Loss of function of one of these genes results in genomic instability leading to increased expression of tumour surface neo-antigens and thereby increases response rates to anti-cancer directed immunotherapies.

Suitably, the algorithm is integrated into a high throughput system used to derive the biomarker data, so that the results are generated. Such systems will comprise a processor and a memory storing instructions to receive the data obtained using the method of the invention, analyse and transform it to produce a 'score' indicative of the presence and specificity of malignancy using the algorithm described above. These results may then be suitably displayed on a graphic interface. In some cases, the memory will comprise a non-transitory computer-readable medium. Such systems and mediums form an aspect of the invention.

Genetic variants detected using the method of the invention may be linked via a suitable bioinformatics platform to a wide range of potential inhibitors of components of the DNA replication initiation pathway including Cdc7 inhibitors from those in clinical trials through to FDA/EMA approved therapies.

Once identified in this way, subjects who are or have been identified as having renal, bladder and/or prostate cancer may be classified depending upon their apparent susceptibility to a certain type of therapy, and may be treated accordingly, using suitable agents. These will be administered in line with normal clinical practice. Thus, conveniently, the method of the invention further comprises generating a customised recommendation for treatment, based upon the results obtained.

The method of the invention addresses the problems and severe limitations of current ELISA based diagnostics. In particular, the method is amenable for full automation. It may be quantitative in particular when using the algorithm described above, and does not require subjective human interpretation by a pathologist. In particular embodiments, the method of the invention does not require the input of a pathologist for manual assessment of biomarkers. The whole test is fully automated and therefore is not subject to inter-observer or intra-observer variability.

Conveniently, the method of the invention provides a comprehensive and integrated readout of all biomarkers listed in Tables 1 to 7 which are linked torenal, bladder and/or prostate cancer. The algorithm, as described above, can be used to integrate all these predictive biomarkers into a Polygenic Detection Score (PDS) as illustrated schematically in Figure 7 hereinafter.

This quantitative nature of the assay means precise cut points may be identified that predict response to specific therapies such as anti-PD-1/PD-L1/PD-L2 directed immunotherapies. By providing a clear quantitative result, any subjectivity and inter-observer variability associated interpretation of conventional assays is avoided.

The method of the invention is able, for example, to assess the complete PD-1/PD-L1 signalling axis in an integrated approach which cannot be achieved using a single IHC biomarker. Using the method of the invention, activation/increased expression of many elements in the PD-1/PD-L1 signalling axis including PD-1, PD-L1, PD-L2, NFATC1, PIK3CA, PIK3CD, PRDM1, PTEN, PTPN11, MTOR, HIF1A, IFN-gamma and FOXO1 may be assessed.

Preferably, the method of the invention includes assessment of oncogenic mutations that are linked to response to anti-PD-1/PD-L1/PD-L2 directed immunotherapies. A broad range of oncogenes are assessed for gene aberration, mutations, fusions and amplification (Tables 5, 6 and 7) with linkage to anti-PD-1/PD-L1 directed immunotherapies. Many of these genes are components of growth signalling networks. Oncogenic activation of these growth signalling networks leads to induction of the PD-1 ligands, PD-L1 and PD-L2.

Thus conveniently, the method of the invention may provide a fully automated test that has been designed to analyse all components involved in the PD-1/PD-L1 immune regulatory anti-renal, bladder and/or prostate cancer response including tumour cells, T immune cells and antigen presenting cells (APSs). This provides a quantitative integrated picture of all components involved in the PD-1/PD-L1/PD-L2 immune regulatory cancer response in terms of all cell types (tumour cell and immune cells) and at all levels of the PD-1/PD-L1 signalling axis.

In addition to a comprehensive analysis such as of the PD-1/PD-L1/PD-L2 signalling axis, the method of the invention has been designed to detect all oncogenic mutations that are indicative of renal, bladder and/or prostate cancer. Integrating information derived from such mutations allows for a customised recommendation for therapy to be prepared. In particular, the association of the PD-1/PD-L1/PD-L2 signalling axis with oncogenic predictors provides the most powerful predictor of response to anti-PD-1/PD-L1/PD-L2 directed immunotherapies. For example detection of (i) mutated and (ii) amplified PD-L1 is also linked to (iii) increased expression of PD-L1. This provides three independent but linked predictors of response to anti-PD-1/PD-L1/PD-L2 directed immunotherapies. By integrating information from the PD-1/PD-L1/PD-L2 signalling axis with oncogenic activation of growth signalling networks, the algorithm described herein is able to precisely identify those patients most likely to respond to anti-PD-1/PD-L1/PD-L2 directed immunotherapies.

The method of the present invention can use high throughput analytical platforms to match patient's tumours to specific targeted therapies, from FDA/EMA approved, ESMO/NCCN guideline references and in all phases of clinical trials worldwide. Examples of linkages between genetic variant and targeted therapies are shown in Figure 2, 4, 5 and 6.

The Thermo Fisher Ion Torrent ^{™} platform has been utilised to develop the assay of the invention. The aim was, for the reasons explained above, to provide a means for not only detecting genito-urinary cancers, but also providing a comprehensive picture of the tumours, to allow appropriate therapies to be prescribed.

Use of a single integrated test as described herein, and using sets of primers (see Example 1, 1.1) spanning the exon/intron boundaries of both immune regulatory genes at multiple loci across the both genes and has been designed in such a way that they are able to amplify the degraded RNA material extracted from routine formalin fixed urine samples. The accurate and precise measurement of these multiple components, such as those of the PD-1/PD-L1/PD-L2 signalling axis allows for the provision of a quantitative integrated profile of this immune regulatory pathway. The output from this assay platform can then be used to provide precise cut offs for these immune regulatory biomarkers which are predictive of therapeutic response to different therapies including in particular anti-PD-1/PD-L1/PD-L2 directed therapies.

The DNA analysis is designed to detect oncogenic mutations and gene copy aberrations which have been identified not only as indicators of malignancy but also predictors of response to specific therapies such as anti- PD-1/PD-L1/PD-L2 directed immunotherapies. In addition to RNA expression analysis of PD-1/PD-L1/PD-L2 signalling molecules, RNA expression analysis can be performed to detect oncogenic fusion transcripts identified as predictors of response to anti- PD-1/PD-L1/PD-L2 directed immunotherapies.

Kits suitable for carrying out the method of the invention may comprise combinations of amplification primers required to detect 3 or more of the biomarkers listed in Tables 1 to 7 and/or the TMB. The kits are not claimed *per se,* but may be used in the practice of the invention. Furthermore, apparatus arranged to carry out the method described above are also novel and form a further aspect of the invention. In particular, apparatus will comprise means for carrying out DNA and/or RNA analyses as described above, linked to a computer programmed to implement the algorithm as described above. A computer or a machine-readable cassette programmed in this way forms yet a further aspect of the invention.

### Detailed Description of the Invention

The invention will now be described by way of example with reference to the accompanying diagrammatic drawings in which:
**Figure 1** shows a schematic diagram illustrating the method of the invention. Tumour cells and/or cell free DNA/RNA are isolated from urine. DNA and RNA is then analysed using next generation sequencing profiling specifically covering cancer genes linked to targeted therapies and immunotherapies. These cancer genes represent companion diagnostics (CDx) to the corresponding targeted therapies/immunotherapies. In addition to providing personalized medicine with regard to treatment selection integrated multiparameter analysis of these companion diagnostic, actionable genetic mutations can also be utilized in cancer detection, screening, analysis of haematuira patients, surveillance and monitoring of therapeutic response.
**Figure 2** shows an example of measurement of Tumour Mutational Burden (TMB). Panel A: shows the TMB readout and Panel B shows bioinformatics linkage with relevant evidence based cognate anti-PD-L1/PD-1 immunotherapies.
**Figure 3** shows analytical validation of the quantative measurements of mRNA levels by NGS in FFPE samples using PD-L1 expression as an example. Reads per million (RPM) counts is used as a surrogate measure of mRNA gene expression. Four cell line controls stably expressing variable levels of PD-L1 assessed by PD-L1 protein were selected representing tumour proportions score of 0%, 25%, 75% and 100% as assessed at the protein level by immunocytpchemistry. The RPM counts are shown for two primer sets spanning exon/intron boundaries for the PD-L1 gene.
   A) shows RPM counts from the two different amplicons targeting the PD-L1 gene.
   B) shows PD-L1 RPM counts (mRNA) generated by the method of the present invention compared to PD-L1 protein expression assessed by IHC.
   C) shows photomicrographs of four cell line controls immunohistochemically stained with an antibody against PD-L1 and expressing different levels of PD-L1 protein. The data shows that the method of the present invention provides an accurate quantative assessment of mRNA expression when applied to routine formalin fixed paraffin wax embedded samples. Notably the RPM shows a rapid increase in parallel with protein expression as measured by ICC across cut point values of 1%, 10%, 25% and 50%. These are the clinically important cut points defined by a number of approved ICC Cdx PD-L1 assays for the identification of responders to anti-PD-L1/PD-1 directed immunotherapies (eg VENTANA PD-L1 (SP263) Assay, VENTANA PD-L1 (SP142) Assay, Dako PD-L1 IHC 28-8 pharmDx, Dako PD-L1 IHC 22C3 pharmDx). Application of this method using multiple primer sets spanning exon/intron boundaries across all genes subject to expression analysis (see Tables 1, 3 and 5 is incorporated into the method of the present invention.
**Figure 4** shows detection of Single Nucleotide Variants (SNVs) and linked cognate targeted therapies. In this tumour sample two actionable SNVs were detected in the PIK3CA oncogene and the p53 tumour suppressor gene (Panel A). Panel B shows identified variant details and the bioinformatics linked cognate targeted therapies are shown in Panel C.
**Figure 5** shows detection of oncogenic fusion genes and linked cognate targeted therapies. In this tumour sample an oncogenic fusion involving the BRAF gene and the mitochondrial ribosomal gene MRPS33 were detected (Panel A). The bioinformatics linked cognate targeted therapies are shown in Panel B (same Key as Figure 4).
**Figure 6** shows detection of gene amplification variants and linked cognate targeted therapies. In this tumour sample gene amplification was detected in relation to two cancer driver genes AKT2 and CCNE1 (Panel A and B).The bioinformatics linked cognate targeted therapies are shown in Panel C (same Key as Figure 4).
**Figure 7** is is a schematic diagram illustrating a method embodying the invention including a polygenic prediction score algorithm used to interpret the results and provide an indication of malignancy (same Key as Figure 4).

### Example 1

### Detection of Genetic Biomarkers

### 1.1 Overview of primer design:

Primers for detecting each of the biomarkers listed in Tables 1 to 7 were designed in accordance with conventional practice using techniques known to those skilled in the art. In general, primer of 18-30 nucleotides in length are optimal with a melting temperature (Tₘ) between 65°C- 75°C. The GC content of the primers should be between 40 - 60%, with the 3' of the primer ending in a C or G to promote binding. The formation of secondary structures within the primer itself is minimised by ensuring a balanced distribution of GC-rich and AT-rich domains. Intra/inter - primer homology should be avoided for optimal primer performance.

### 1.1.1 Primers for copy number detection:

Primers were designed, as discussed in 1.1, to span regions in the genes listed in Tables 4 and 6. Several amplicons per gene were designed. The depth of coverage is measured for each of these amplicons. The copy number amplification and deletion algorithm is based on a hidden Markov model (HMM). Prior to copy number determination, read coverage is corrected for GC bias and compared to a preconfigured baseline.

### 1.1.2 Primer for hotspot detection:

Primers were designed, as discussed in 1.1, to target specific regions prone to oncogenic somatic mutations as listed in Table 2 and in consideration with the general points discussed above.

### 1.1.3 Primers for RNA expression analysis:

Extracted RNA is processed via RT-PCR to create complementary DNA (Cdna) which is then amplified using primers designed, as discussed in 1.1. Multiple primer sets were designed to span the exon/intron boundaries across all genes subject to expression analysis as listed Table 1 and 5

### 1.1.4 Primers for RNA fusion detection:

A pair of targeted exon-exon breakpoint assay primers were designed, as discussed in 1.1, for each fusion listed in Tables 3 and 5. Primers flanking the fusion breakpoint generate specific fusion amplicons which are aligned to the reference sequence allowing for identification of fusion genes. Expression imbalance assays enable the equivalent expression levels to be monitored in normal samples, with an imbalance between the 5' and 3' assays indicating samples have a fusion breakpoint.

### 1.2 DNA and RNA extraction

DNA and RNA was extracted from a formalin fixed urine sample. Two xylene washes were performed by mixing 1ml of xylene with the sample. The samples were centrifuged and xylene removed. This was followed by 2 washes with 1ml of pure ethyl alcohol. After the samples were air-dried, 25µl of digestion buffer, 75µl of nuclease free water and 4µl of protease were added to each sample. Samples were then digested at 55°C for 3 hours followed by 1 hour digestion at 90°C.

120µl of Isolation additive was mixed with each sample and the samples added to filter cartridges in collection tubes and centrifuged. The filters were moved to new collection tubes and kept in the fridge for DNA extraction at a later stage. The flow-through was kept for RNA extraction and 275µl of pure ethyl alcohol was added and the sample moved to a new filter in a collection tube and centrifuged. After a wash of 700µl of Wash 1 buffer the RNA was treated with DNase as follows; a DNase mastermix was prepared using 6µl of 10X DNase buffer, 50µl of nuclease free water and 4µl of DNase per sample. This was added to the centre of each filter and incubated at room temperature for 30 minutes.

After the incubation 3 washes were performed using Wash 1, then Wash 2/3 removing the wash buffer from the collection tubes after each centrifugation. The filters were moved to a new collection tube and the elution solution (heated to 95°C) was added to each filter and incubated for 1 minute. After centrifuging the sample, the filter was discarded and the RNA collected in the flow through moved to a new low bind tube.

The DNA in the filters were washed with Wash 1 buffer, centrifuged and flow through discarded. The DNA was treated with RNase (50µl nuclease water and 10µl RNase) and incubated at room temperature for 30 minutes. As above with the RNA, three washes were completed and the samples eluted in elution solution heated at 95°C.

### 1.3 DNA and RNA measurement

The quantity of DNA and RNA from the extracted samples were measured using the Qubit^{®} 3.0 fluorometer and the Qubit^{®} RNA High Sensitivity Assay kit (CAT: Q32855) and Qubit^{®} dsDNA High Sensitivity Assay kit (Cat:Q32854). 1µl of RNA/DNA combined with 199µl of combined HS buffer and reagent were used in Qubit^{®} assay tubes for measurement. 10µl of standard 1 or 2 were combined with 190µl of the buffer and reagent solution for the controls.

### 1.4 Library preparation

RNA samples were diluted to 5ng/µl if necessary and reverse transcribed to cDNA in a 96 well plate using the SuperScript VILO cDNA synthesis kit (CAT 11754250). A mastermix of 2µl of VILO, 1µl of 10 X SuperScript III Enzyme mix and 5µl of nuclease free water was made for all of the samples. 8µl of the MasterMix was used along with 2µl of the RNA in each well of a 96 well plate. The following program was run:

| **Temperature** | **Time** |
|---|---|
| 42ºC | 30 min |
| 85ºC | 5 min |
| 10ºC | Hold |

Amplification of the cDNA was then performed using 4µl of 6 RNA primers covering multiple exon-intron loci across the gene, 4µl of AmpliSeq HiFi^{∗1} and 2µl of nuclease free water into each sample well. The plate was run on the thermal cycler for 30 cycles using the following program:

| **Stage** | **Step** | **Temperature** | **Time** |
|---|---|---|---|
| Hold | Activate the enzyme | 99ºC | 2 min |
| Cycle (30 cycles) | Denature | 99ºC | 15 sec |
| | Anneal and extend | 60ºC | 4 min |
| Hold | - | 10ºC | Hold |

DNA samples were diluted to 5ng/µl and added to AmpliSeq Hifi^{∗1}, nuclease free water and set up using two DNA primer pools (5µl of pool 1 and 5µl of pool 2) in a 96 well plate. The following program was run on the thermal cycler:

| **Stage** | **Step** | **Temperature** | **Time** |
|---|---|---|---|
| Hold | Activate the enzyme | 99ºC | 2 min |
| Cycle (18 cycles) | Denature | 99ºC | 15 sec |
| | Anneal and extend | 60ºC | 4 min |
| Hold | - | 10ºC | Hold (up to 16 hours) |

Following amplification, the amplicons were partially digested using 2µl of LIB Fupa^{∗1}, mixed well and placed on the thermal cycler on the following program:

| **Temperature** | **Time** |
|---|---|
| 50ºC | 10 min |
| 55ºC | 10 min |
| 60ºC | 20 min |
| 10ºC | Hold (for up to 1 hour) |

4µl of switch solution^{∗1}, 2µl of diluted Ion XPRESS Barcodes 1-16 (CAT: 4471250) and 2µl of LIB DNA ligase^{∗1} were added to each sample, mixing thoroughly in between addition of each component. The following program was run on the thermal cycler:

| **Temperature** | **Time** |
|---|---|
| 22ºC | 30 min |
| 72ºC | 10 min |
| 10ºC | Hold (for up to 1 hour) |

The libraries were then purified using 30µl of Agencourt AMPure XP (Biomeck Coulter cat: A63881) and incubated for 5 minutes. Using a plate magnet, 2 washes using 70% ethanol were performed. The samples were then eluted in 50µl TE.

### 1.5 qPCR

The quantity of library was measured using the Ion Library TaqMan quantitation kit (cat: 4468802). Four 10-fold serial dilutions of the *E.coli* DH10B Ion control library were used (6.8pmol, 0.68pmol, 0.068pmol and 0.0068pmol) to create the standard curve. Each sample was diluted 1/2000, and each sample, standard and negative control were tested in duplicate. 10µl of the 2X TaqMan mastermix and 1µl of the 20X TaqMan assay were combined in a well of a 96 well fast thermal cycling plate for each sample. 9µl of the 1/2000 diluted sample, standard or nuclease free water (negative control) were added to the plate and the qPCR was run on the ABI StepOnePlus^{™} machine (Cat: 4376600) using the following program:

| **Stage** | **Temperature** | **Time** |
|---|---|---|
| Hold (UDG incubation) | 50ºC | 2 min |
| Hold (polymerase activation) | 95ºC | 20 sec |
| Cycle (40 cycles) | 95ºC | 1 sec |
| | 60ºC | 20 sec |

Samples were diluted to 100pmol using TE and 10µl of each sample pooled to either a DNA tube or RNA tube. To combine the DNA and RNA samples, a ratio of 80:20 DNA:RNA was used.

### 1.6 Template preparation

The Ion One Touch^{™} 2 was initialized using the Ion S5 OT2 solutions and supplies^{∗2} and 150µl of breaking solution^{∗2} was added to each recovery tube. The pooled RNA samples were diluted further in nuclease free water (8µl of pooled sample with 92µl of water) and an amplification mastermix was made using the Ion S5 reagent mix^{∗2} along with nuclease free water, ION S5 enzyme mix^{∗2}, Ion sphere particles (ISPs) ^{∗2} and the diluted library. The mastermix was loaded into the adapter along with the reaction oil^{∗2}. The instrument was loaded with the amplification plate, recovery tubes, router and amplification adapter loaded with sample and amplification mastermix.

### 1.7 Enrichment

For the enrichment process, melt off was made using 280µl of Tween^{∗2} and 40µl of 1M Sodium Hydroxide. Dynabeads^{®} MyOne^{™} Streptavidin C1 (CAT:65001) were washed with the OneTouch wash solution^{∗2} using a magnet. The beads were suspended in 130µl of MyOne bead capture solution^{∗2}. The ISPs were recovered by removing the supernatant, transferring to a new low bind tube and subsequently washed in 800µl of nuclease free water. After centrifuging the sample and removing the supernatant of water, 20µl of template positive ISPs remained. 80µl of ISP resuspension solution^{∗2} was added for a final volume of 100µl.

A new tip, 0.2ml tube and an 8 well strip was loaded on the OneTouch^{™} ES machine with the following:
Well 1: 100µl of template positive ISPs
Well 2: 130µl of washed Dynabeads^{®} MyOne^{™} streptavidin C1 beads, resuspended in MyOne bead capture
Well 3: 300µl of Ion OneTouch ES Wash solution^{∗2}
Well 4: 300µl of Ion OneTouch ES Wash solution
Well 5: 300µl of Ion OneTouch ES Wash solution
Well 6: Empty
Well 7: 300µl of melt off
Well 8: Empty

Following the run which takes approximately 35 minutes, the enriched ISPs were centrifuged, the supernatant removed and washed with 200µl of nuclease free water. Following a further centrifuge step and supernatant removal, 10µl of ISPs remained. 90µl of nuclease free water was added and the beads were resuspended.

### 1.8 Sequencing

The Ion S5 system^{™} (Cat: A27212) was initialized using the Ion S5 reagent cartridge, Ion S5 cleaning solution and Ion S5 wash solutions^{∗2}.

5µl of Control ISPs^{∗2} were added to the enriched sample and mixed well. The tube was centrifuged and the supernatant removed to leave the sample and control ISPs. 15µl of Ion S5 annealing buffer^{∗2} and 20µl of sequencing primer^{∗2} were added to the sample. The sample was loaded on the thermal cycler for primer annealing at 95°C for 2 minutes and 37°C for 2 minutes. Following thermal cycling, 10µl of Ion S5 loading buffer^{∗2} was added and the sample mixed.

50% annealing buffer was made using 500µl of Ion S5 annealing buffer^{∗2} and 500µl of nuclease free water^{∗2}.

The entire sample was then loaded into the loading port of an Ion 540^{™} chip (Cat: A27766) and centrifuged in a chip centrifuge for 10 minutes.

Following this, 100µl of foam (made using 49µl of 50% annealing buffer and 1µl of foaming solution^{∗2}) was injected into the port followed by 55µl of 50% annealing buffer into the chip well, removing the excess liquid from the exit well. The chip was centrifuged for 30 seconds with the chip notch facing out. This foaming step was repeated.

The chip was flushed twice using 100µl of flushing solution (made using 250µl of isopropanol and 250µl of Ion S5 annealing buffer) into the loading port, and excess liquid removed from the exit well. 3 flushes with 50% annealing buffer into the loading port were then performed. 60µl of 50% annealing buffer was combined with 6µl of Ion S5 sequencing polymerase^{∗2}. 65µL of the polymerase mix was then loaded into the port, incubated for 5 minutes and loaded on the S5 instrument for sequencing which takes approximately 3 hours and 16 hours for data transfer.
*1 From the Ion Ampliseq^{™} library 2.0 (Cat:4480441)
*2 From the Ion 540^{™} OT2 kit (Cat: A27753)

### 1.9 Data Analysis

### 1.9.1 DNA CNV analysis:

Copy number variations (CNVs) represent a class of variation in which segments of the genome have been duplicated (gains) or deleted (losses). Large, genomic copy number imbalances can range from sub-chromosomal regions to entire chromosomes.

Raw data were processed on the Ion S5 System and transferred to the Torrent Server for primary data analysis performed using the Oncomine Comprehensive Assay Baseline v2.0. This plug-in is included in Torrent Suite Software, which comes with each Ion Torrent^{™} sequencer. Copy number amplification and deletion detection was performed using an algorithm based on a hidden Markov model (HMM). The algorithm uses read coverage across the genome to predict the copy-number. Prior to copy number determination, read coverage is corrected for GC bias and compared to a preconfigured baseline.

The median of the absolute values of all pairwise differences (MAPD) score is reported per sample and is used to assess sample variability and define whether the data are useful for copy number analysis. MAPD is a per-sequencing run estimate of copy number variability, like standard deviation (SD). If one assumes the log2 ratios are distributed normally with mean 0 against a reference a constant SD, then MAPD/0.67 is equal to SD. However, unlike SD, using MAPD is robust against high biological variability in log2 ratios induced by known conditions such as cancer. Samples with an MAPD score above 0.5 should be carefully reviewed before validating CNV call.

The results from copy number analysis after normalisation can be visualised from the raw data.

Somatic CNV detection provides Confidence bounds for each Copy Number Segment. The Confidence is the estimated percent probability that Copy Number is less than the given Copy Number bound. A lower and upper percent and the respective Copy Number value bound are given for each CNV. Confidence intervals for each CNV are also stated, and amplifications of a copy number > 6 with the 5% confidence value of ≥4 after normalization and deletions with 95% Cl ≤1 are classified as present.

### 1.9.2 DNA hotspot analysis:

Raw data were processed on the Ion S5 System and transferred to the Torrent Server for primary data analysis performed using the custom workflow. Mapping and alignment of the raw data to a reference genome is performed and then hotspot variants are annotated in accordance with the BED file. Coverage statistics and other related QC criteria are defined in a vcf file which includes annotation using a rich set of public sources. Filtering parameters can be applied to identify those variants passing QC thresholds and these variants can be visualised on IGV. In general, the rule of classifying variants with >10% alternate allele reads, and in >10 unique reads are classified as 'detected'.

Several in-silico tools are utilised to assess the pathogenicity of identified variants these include PhyloP, SIFT, Grantham, COSMIC and PolyPhen-2.

### 1.9.3 RNA expression analysis:

Raw data were processed on the Ion S5 System and transferred to the Torrent Server for primary data analysis performed using the AmpliSeqRNA plug-in. This plug-in is included in Torrent Suite Software, which comes with each Ion Torrent^{™} sequencer. The AmpliSeqRNA plugin uses the Torrent Mapping Alignment Program (TMAP). TMAP is optimized for Ion Torrent^{™} sequencing data for aligning the raw sequencing reads against a custom reference sequence set containing all transcripts targeted by the AmpliSeq kit. The assay specific information is contained within a bespoke BED file. To maintain specificity and sensitivity, TMAP implements a two-stage mapping approach. First, four alignment algorithms, BWA-short, BWA-long, SSAHA, and Super-maximal Exact Matching we employed to identify a list of Candidate Mapping Locations (CMLs). A further aligning process is performed using the Smith Waterman algorithm to find the final best mapping. As part of the ampliSeqRNA plugin, raw read counts of the targeted genes is performed using samtools (samtools view -c -F 4 -L bed_file bam_file). Ion AmpliSeq RNA normalization for a given sample is automatically calculated by the plug-in as the number of reads mapped per gene per million reads mapped or RPM. This figure is then log2 -transformed normalized reads per million, (nRPM).

The bespoke BED file is a formatted to contain the nucleotide positions of each amplicon per transcript in the mapping reference. Reads aligning to the expected amplicon locations and meeting filtering criteria such as minimum alignment length are reported as percent "valid" reads. "Targets Detected" is defined as the number of amplicons detected (≥10 read counts) as a percentage of the total number of targets.

After mapping, alignment and normalization, The AmpliSeqRNA plug-in provides data on QC metrics, visualization plots, and normalized counts per gene that corresponds to gene expression information that includes a link to a downloadable file detailing the read counts per gene in a tab-delimited text file. The number of reads aligning to a given gene target represents an expression value referred to as "counts". This Additional plug-in analyses include output for each barcode of the number of genes (amplicons) with at least 1, 10, 100, 1,000, and 10,000 counts to enable determination of the dynamic range and sensitivity per sample.

A summary table of the above information, including mapping statistics per barcode of total mapped reads, percentage on target, and percentage of panel genes detected ("Targets Detected") is viewable in Torrent Suite Software to quickly evaluate run and library performance.

### Example 2

### Analysis of tumour mutational burden.

### 2.0 DNA measurement

DNA from a urine sample was quantified post extraction following the protocol in section 1.3 above.

### 2.1 Library preparation

DNA samples were diluted to 5ng/µl and added to 5X Ion AmpliSeq Hifi (from the From the Ion AmpliSeq^{™} library kit plus (4488990), nuclease free water and set up using two DNA primer pools (5µl of pool 1 and 5µl of pool 2) in a 96 well plate. The following program was run on the thermal cycler:

| **Stage** | **Step** | **Temperature** | **Time** |
|---|---|---|---|
| Hold | Activate the enzyme | 99°C | 2 min |
| Cycle (15) | Denature | 99°C | 15 sec |
| | Anneal and extend | 60°C | 16 min |
| Hold | - | 10°C | Hold |

Following amplification, the amplicons were partially digested using 2µl of LIB FuPa (From the Ion 540^{™} OT2 kit (Cat: A27753)), mixed well and placed on the thermal cycler on the following program:

| **Temperature** | **Time** |
|---|---|
| 50°C | 20 min |
| 55°C | 20 min |
| 60°C | 20 min |
| 10°C | Hold (for up to 1 hour) |

4µl of switch solution^{∗3}, 2µl of diluted Ion XPRESS Barcodes 1-16 (Cat: 4471250) and 2µl of LIB DNA ligase (From the Ion Ampliseq^{™} library kit plus (4488990)) were added to each sample, mixing thoroughly in between addition of each component. The following program was run on the thermal cycler:

| **Temperature** | **Time** |
|---|---|
| 22°C | 30 min |
| 68°C | 5 min |
| 72°C | 5 min |
| 10°C | Hold (for up to 24 hour) |

### 2.3 Purification

Libraries were purified as in section 1.3 using 45µl of Agencourt AMPure XP (Biomeck Coulter cat: A63881).

### 2.4 q-PCR

The quantity of library was measured using the Ion Library TaqMan quantitation kit (cat: 4468802). Three 10-fold serial dilutions of the *E.coli* DH10B Ion control library were used (6.8pmol, 0.68pmol and 0.068pmol) to create the standard curve. Each sample was diluted 1/500 and each sample, standard and negative control were tested in duplicate. 10µl of the 2X TaqMan mastermix and 1µl of the 20X TaqMan assay were combined in a well of a 96 well fast thermal cycling plate for each sample. 9µl of the 1/500 diluted sample, standard or nuclease free water (negative control) were added to the plate and the qPCR was run on the ABI StepOnePlus^{™} machine (Cat: 4376600) using the program listed in section 1.5.

Samples were diluted to 100pMol using the results from the q-PCR and pooled ready for template preparation. Following this, template preparation, enrichment of the sample and sequencing were performed as written in sections 1.6, 1.7 and 1.8 respectively.

### Example 3

### Application of Polygenic Detection Score (PDS) algorithm to results

Case 1. Results obtained from a sample from a patient with macroscopic haematuria.

Assay results:
A. SNV Hotspot mutation: BRAF (PDS= 1)
B. CNV: PD-L1 amplificationT, JAK2 amplification (PDS= 2+2=4)
C. TMB= 5mut/MB (PDS=1)
D. Gene fusions: no fusion detected (PDS= 0)
E. Gene expression
   a. High PD-L1 expression (PDS= 3)
   b. Proliferation signature: high MCM2-3-5, high GEMININ, High PLK1, High FOXM1, High BUB1 (PDS= 2)

### PDS Algorithm score= 11

Indicative of malignancy and potential response to anti-PD-L1 inhibitors (e.g Durvalumab, pembrolizumab) and BRAF inhibitors.

### Cell cycle regulated genes - Table 1

### Detection of increased expression of these proliferation linked genes is indicative of the presence of genito-urinary tract cancer

| **Proliferation markers** | |
|---|---|
| BUB1 | MELK |
| CCNB2 | MKI67 |
| CD3D | TOP2A |
| CD3E | MCM2 |
| CD3G | MCM3 |
| CDK1 | MCM5 |
| CDKN3 | GEMININ |
| FOXM1 | PLK1 |
| KIAA0101 | MRE11A |
| MAD2L1 | |

### Actionable mutations in oncogenes and tumour suppressors including fusion genes (Table 2, 3 and 4)

**Table 2: Actionable genetic variants - point mutations in targeted Oncogenic hotspots and full length tumour suppressor gene sequencing.**

| | | |
|---|---|---|
| ABL1 mutation | ERBB2 L869R mutation | KRAS A59 mutation |
| ABL1-BCR E255K mutation | ERBB2 mutation | KRAS aberration |
| ABL1-BCR E255V mutation | ERBB2 mutation status | KRAS activating mutation |
| ABL1-BCR F317C mutation | ERBB2 positive | KRAS exon 2 mutation |
| ABL1-BCR F317I mutation | ERBB2 S310F mutation | KRAS exon 3 mutation |
| ABL1-BCR F317L mutation | ERBB2 status | KRAS exon 4 mutation |
| ABL1-BCR F317V mutation | ERBB2 V659E mutation | KRAS G12 mutation |
| ABL1-BCR F359C mutation | ERBB2 V777L mutation | KRAS G12V mutation |
| ABL1-BCR F359I mutation | ERBB3 mutation | KRAS G13 mutation |
| ABL1-BCR F359V mutation | ERBB4 mutation | KRAS K117 mutation |
| ABL1-BCR KD mutation | ERCC1 mutation | KRAS mutation |
| ABL1-BCR T315A mutation | ERCC2 mutation | KRAS mutation status |
| ABL1-BCR T315I mutation | FANC mutation | KRAS positive |
| ABL1-BCR V299L mutation | FANCA mutation | KRAS Q61 mutation |
| ABL1-BCR Y253H mutation | FANCC mutation | MET activating mutation |
| AKT mutation | FANCD2 mutation | MET mutation |
| AKT1 activating mutation | FANCF mutation | MLH1 mutation |
| AKT1 mutation | FANCG mutation | MMR pathway |
| AKT2 aberration | FBXW7 mutation | MRE11 mutation |
| AKT2 activating mutation | FGF aberration | MSH2 mutation |
| AKT2 E17K mutation | FGF19 aberration | MSH6 mutation |
| AKT2 mutation | FGFR aberration | MTOR aberration |
| AKT3 activating mutation | FGFR activating mutation | MTOR activating mutation |
| AKT3 E17K mutation | FGFR mutation | MTOR mutation |
| AKT3 mutation | FGFR pathway | NBN mutation |
| ALK aberration | FGFR1 aberration | NF1 aberration |
| ALK mutation | FGFR1 K656E mutation | NF1 mutation |
| ATM aberration | FGFR1 mutation | NF2 aberration |
| ATM mutation | FGFR1 N546K mutation | NF2 mutation |
| ATR mutation | FGFR1 T141A mutation | NRAS A146 mutation |
| AXL mutation | FGFR2 aberration | NRAS A59 mutation |
| BAP1 mutation | FGFR2 C382R mutation | NRAS aberration |
| BLM mutation | FGFR2 G380R mutation | NRAS activating mutation |
| BRAF aberration | FGFR2 K659E mutation | NRAS exon 2 mutation |
| BRAF activating mutation | FGFR2 mutation | NRAS exon 3 mutation |
| BRAF mutation | FGFR2 N549K mutation | NRAS exon 4 mutation |
| BRAF mutation status | FGFR2 P253R mutation | NRAS G12 mutation |
| BRAF V600 mutation | FGFR2 positive | NRAS G12V mutation |
| BRAF V600D mutation | FGFR2 S252W mutation | NRAS G13 mutation |
| BRAF V600E mutation | FGFR2 Y375C mutation | NRAS K117 mutation |
| BRAF V600E mutation status | FGFR3 A391E mutation | NRAS mutation |
| BRAF V600K mutation | FGFR3 aberration | NRAS mutation status |
| BRAF V600R mutation | FGFR3 activating mutation | NRAS Q61 mutation |
| BRCA mutation | FGFR3 F384L mutation | NTRK aberration |
| BRCA1 aberration | FGFR3 F386L mutation | NTRK activating mutation |
| BRCA1 mutation | FGFR3 G370C mutation | NTRK mutation |
| BRCA2 aberration | FGFR3 G372C mutation | NTRK1 aberration |
| BRCA2 mutation | FGFR3 G380R mutation | NTRK1 positive |
| BRIP1 mutation | FGFR3 G382R mutation | NTRK2 aberration |
| CBL mutation | FGFR3 G697C mutation | NTRK2 positive |
| CD274 positive | FGFR3 K650E mutation | NTRK3 aberration |
| CDK12 mutation | FGFR3 K650M mutation | NTRK3 positive |
| CDK4 mutation | FGFR3 K650Q mutation | PALB2 mutation |
| DDR2 S768R mutation | FGFR3 K650T mutation | PDGFR aberration |
| CDK6 mutation | FGFR3 K652E mutation | PDGFR mutation |
| EGFR A289V mutation | FGFR3 K652M mutation | PDGFRA aberration |
| CDKN2A mutation | FGFR3 K652T mutation | PDGFRA D842 mutation |
| CDKN2A negative | FGFR3 mutation | PDGFRA D842V mutation |
| CDKN2A status | FGFR3 R248C mutation | PDGFRA mutation |
| CDKN2B mutation | FGFR3 S249C mutation | PDGFRB aberration |
| CHEK1 mutation | FGFR3 S371C mutation | PDGFRB mutation |
| CHEK2 mutation | FGFR3 S373C mutation | PI3K activating mutation |
| CSF1R aberration | FGFR3 Y373C mutation | PI3K/AKT/MTOR mutation |
| CSF1R mutation | FGFR3 Y375C mutation | PI3K/AKT/MTOR pathway |
| DDR2 I638F mutation | FGFR4 aberration | PIK3CA aberration |
| DDR2 L239R mutation | FGFR4 mutation | PIK3CA activating mutation |
| DDR2 mutation | FLT1 mutation | PIK3CA exon 20 mutation |
| EGFR activating mutation | FLT3 aberration | PIK3CA exon 9 mutation |
| EGFR exon 18 activating mutation | FLT3 activating mutation | PIK3CA mutation |
| EGFR exon 18 mutation | FLT3 D835 mutation | PIK3CA mutation status |
| EGFR exon 19 activating mutation | FLT3 I836 mutation | PIK3CB mutation |
| EGFR exon 19 deletion | FLT3 ITD mutation | PIK3CG mutation |
| EGFR exon 19 insertion | FLT3 mutation | PIK3R1 aberration |
| EGFR exon 19 mutation | FLT3 positive | PIK3R1 mutation |
| EGFR exon 19 sensitizing mutation | FLT3 TKD mutation | PIK3R2 activating mutation |
| EGFR exon 20 activating mutation | FLT4 mutation | PMS2 mutation |
| EGFR exon 20 deletion | G1/S cell cycle pathway | POLE aberration |
| EGFR exon 20 insertion | GNA11 mutation | POLE codons 268-471 mutation |
| EGFR exon 20 mutation | GNAQ mutation | POLE mutation |
| EGFR exon 20 resistance mutation | HRAS activating mutation | PTCH1 mutation |
| EGFR exon 21 activating mutation | HRAS G12 mutation | PTEN aberration |
| EGFR exon 21 mutation | HRAS G12V mutation | PTEN mutation |
| EGFR exon 21 sensitizing mutation | HRAS G13 mutation | RAD50 mutation |
| EGFR G598V mutation | HRAS mutation | RAD51 mutation |
| EGFR G719 mutation | HRAS Q61 mutation | RAD51B mutation |
| EGFR G719A mutation | HRR mutation | RAD51C mutation |
| EGFR G719C mutation | HRR pathway | RAD51D mutation |
| EGFR G719S mutation | IDH1 mutation | RAF aberration |
| EGFR L62R mutation | IDH1 R132 mutation | RAF1 mutation |
| EGFR L858 mutation | IDH1 R132H mutation | RARA-PMLfusion |
| EGFR L858R mutation | IDH2 mutation | RAS activating mutation |
| EGFR L861 mutation | IDH2 R140 mutation | RAS mutation |
| EGFR L861Q mutation | IDH2 R140G mutation | RAS/RAF/MEK/ERK mutation |
| EGFR L861R mutation | IDH2 R140L mutation | RAS/RAF/MEK/ERK pathway |
| EGFR mutation | IDH2 R140Q mutation | RB1 mutation |
| EGFR mutation status | IDH2 R140W mutation | RB1 status |
| EGFR P596L mutation | IDH2 R172 mutation | RET aberration |
| EGFR positive | IDH2 R172G mutation | RET activating mutation |
| EGFR R108K mutation | IDH2 R172K mutation | RET mutation |
| EGFR R222C mutation | IDH2 R172M mutation | RET mutation status |
| EGFR resistance mutation | IDH2 R172S mutation | RET positive |
| EGFR S768I mutation | IDH2 R172W mutation | RHEB aberration |
| EGFR T790M mutation | JAK2 V617F mutation | ROS1 aberration |
| EGFR T790M mutation status | KDR aberration | ROS1 mutation |
| EGFR V769L mutation | KDR mutation | ROS1 positive |
| EGFR V774M mutation | KDR positive | SMARCB1 mutation |
| EGFRi sensitizing mutation | KIT aberration | SMO activating mutation |
| ERBB aberration | KIT activating mutation | SMO mutation |
| ERBB2 aberration | KIT D816V mutation | SRC mutation |
| ERBB2 activating mutation | KIT exon 11 mutation | STK11 aberration |
| ERBB2 D769H mutation | KIT exon 13 mutation | STK11 mutation |
| ERBB2 E757A mutation | KIT exon 14 mutation | TP53 mutation |
| ERBB2 exon 19 deletion | KIT exon 17 mutation | TSC mutation |
| ERBB2 exon 20 deletion | KIT exon 18 mutation | TSC1 aberration |
| ERBB2 exon 20 insertion | KIT exon 8 mutation | TSC1 mutation |
| ERBB2 G776V mutation | KIT exon 9 mutation | TSC2 aberration |
| ERBB2 L755A mutation | KIT mutation | TSC2 mutation |
| ERBB2 L755S mutation | KIT resistance mutation | |
| ERBB2 L841V mutation | KRAS A146 mutation | |

**Table 3: Actionable genetic variants - Fusions.**

| | |
|---|---|
| ABL1 fusion | FGFR2-PLPP4 fusion |
| ABL1-BCR fusion | FGFR2-SLC45A3 fusion |
| ALK fusion | FGFR2-TACC2 fusion |
| ALK-CLIP4 fusion | FGFR2-UBQLN1 fusion |
| ALK-CLTC fusion | FGFR2-VCL fusion |
| ALK-EML4 fusion | FGFR3 fusion |
| ALK-KIF5B fusion | FGFR3-BAIAP2L1 fusion |
| ALK-NPM1 fusion | FGFR3-NSD2 fusion |
| ALK-RANBP2 fusion | FGFR3-TACC3 fusion |
| ALK-STRN fusion | FLT3 amplification |
| ALK-TFG fusion | FLT3 fusion |
| AXL fusion | KRAS fusion |
| BRAF fusion | MET exon 14 mutation |
| BRCA1 fusion | MET exon 14 skipping mutation |
| BRCA2 fusion | MET fusion |
| EGFR vIII | NTRK fusion |
| FGFR fusion | NTRK1 fusion |
| FGFR1 fusion | NTRK2 fusion |
| FGFR1-TACC1 fusion | NTRK3 fusion |
| FGFR2 fusion | NUTM1 fusion |
| FGFR2-AFF3 fusion | PDGFRA fusion |
| FGFR2-BICC1 fusion | PDGFRB fusion |
| FGFR2-CASP7 fusion | RAF1 fusion |
| FGFR2-CCAR1 fusion | RET fusion |
| FGFR2-CCDC6 fusion | RET-KIF5B fusion |
| FGFR2-CFAP58 fusion | ROS1 fusion |
| FGFR2-MCU fusion | RSPO3 fusion |
| FGFR2-OFD1 fusion | |

**Table 4: Actionable genetic variants - copy number changes.**

| | |
|---|---|
| AKT1 amplification | FGFR2 amplification |
| AKT2 amplification | FGFR3 amplification |
| ALK amplification | FGFR4 amplification |
| ATM deletion | KIT amplification |
| AXL amplification | KRAS amplification |
| BRAF amplification | MET amplification |
| BRCA1 deletion | MYC amplification |
| BRCA2 deletion | MYCL amplification |
| CCND1 amplification | MYCN amplification |
| CCND2 amplification | NTRK amplification |
| CCND3 amplification | PDGFRA amplification |
| CCNE1 amplification | PDGFRB amplification |
| CD274 amplification | PIK3CA amplification |
| CDK4 amplification | PIK3CB amplification |
| CDK6 amplification | PTCH1 deletion |
| CDKN2A deletion | PTEN deletion |
| EGFR amplification | RAS amplification |
| ERBB2 amplification | RB1 deletion |
| ERBB2 overexpression | SMARCB1 deletion |
| FGF amplification | TP53 deletion |
| FGF19 amplification | TSC1 deletion |
| FGFR amplification | TSC2 deletion |
| FGFR1 amplification | |

### Signalling networks linked to the PD-L1 immune checkpoint Table 5, 6 and 7

**Table 5: RNA analysis - expression and fusion targets**

| | | | |
|---|---|---|---|
| CD274 (PDL1) | NKG7 | CDKN3 | >500nRPM |
| PDCD1LG2 (PDL2, CD273) | PTPRC | FOXM1 | >500nRPM |
| PDCD1 (PD1, CD279) | SRGN | KIAA0101 | >500nRPM |
| CXCL9 | AIF1 | MAD2L1 | >500nRPM |
| FOXO1 | CD14 | MELK | >500nRPM |
| HIF1A | CD16 | MKI67 | >500nRPM |
| MTOR | CD163 | TOP2A | >500nRPM |
| NFATC1 | CD68 | MCM2 | >500nRPM |
| PIK3CA | HLA-DQB1 | MCM3 | >500nRPM |
| PIK3CD | HLA-DRB1 | MCM5 | >500nRPM |
| PRDM1 (Blimp1) | HLA-DPA1 | GEMININ | >500nRPM |
| PTEN | HLA-DRA | PLK1 | >500nRPM |
| PTPN11 | CTLA4 | MRE11A | >500nRPM |
| CD19 | IDO1 | COL1A1 | >500nRPM |
| CD38 | CD3E | COL1A2 | >500nRPM |
| CD79A | CD40 (TNFRSF5) | COL3A1 | >500nRPM |
| NTN3 | TNFRSF9 (CD137) | COL5A1 | >500nRPM |
| POU2AF1 | TNFRSF17 | COL5A2 | >500nRPM |
| SKAP2 | LAG3 | COL6A3 | >500nRPM |
| MZB1 | HAVCR2 (TIM3) | MMP2 | >500nRPM |
| TNFRSF17 | CD47 | POSTN | >500nRPM |
| CD3 | TNFRSF4 (OX40) | SPARC | >500nRPM |
| CD4 | TNFRSF18 (GITR) | VCAN | >500nRPM |
| CD8 | CD276 (B7-H3) | ALK | >500nRPM |
| CD56 | TIGIT | FGFR1 | >500nRPM |
| CD15 | BUB1 | FGFR2 | >500nRPM |
| CCL5 | CCNB2 | FGFR3 | >500nRPM |
| CD37 | CD3D | MET | >500nRPM |
| CD53 | CD3E | ROS1 | >500nRPM |
| CTSS | CD3G | NTRK1-3 | >500nRPM |
| FYB | CDK1 | RET | >500nRPM |

**Table 6: Copy number gene targets**

| |
|---|
| CD274 (PD-L1) |
| PDCD1LG2 (PD-L2; CD273) |
| PDCD1 (PD1; CD279) |
| ERBB2 |
| FGFR1 |
| FGFR2 |
| FGFR3 |
| FGFR4 |
| MET |

**Table 7: DNA hotspot, and full gene sequencing targets**

| **Hotspot genes** | **Full length gene targets** |
|---|---|
| ALK | JAK1 |
| BRAF exon 15 | JAK2 |
| EGFR exon 18 | TP53 |
| EGFR exon 19 activating mutation | MLH1 |
| EGFR exon 19 deletion | MSH2 |
| EGFR exon 19 insertion | MSH6 |
| EGFR exon 19 sensitizing mutation | PMS2 + promoter |
| EGFR exon 20 activating mutation | POLE |
| EGFR exon 20 insertion | POLD1 |
| EGFR exon 20 mutation | |
| EGFR exon 21 activating mutation | |
| EGFR exon 21 sensitizing mutation | |
| EGFR G719 mutation | |
| EGFR L858R mutation | |
| EGFR L861 mutation | |
| EGFR S768 mutation | |
| EGFR T790M mutation | |
| KIT activating mutation | |
| KIT resistance mutation | |
| KRAS activating mutation | |
| MET activating mutation | |
| NRAS activating mutation | |
| PMS2 promoter mutations | |

**Table 8. Genes targeted got analysis of Tumour Mutational Burden (TMB).**

| | | | | | | | |
|---|---|---|---|---|---|---|---|
| ABL1 | CCND1 | EP400 | GATA2 | LAMP1 | MYH11 | PLAG1 | SMARCA4 |
| ABL2 | CCND2 | EPHA3 | GATA3 | LCK | MYH9 | PLCG1 | SMARCB1 |
| ACVR2A | CCNE1 | EPHA7 | GDNF | LIFR | NBN | PLEKHG5 | SMO |
| ADAMTS20 | CD79A | EPHB1 | GNA11 | LPHN3 | NCOA1 | PML | SMUG1 |
| AFF1 | CD79B | EPHB4 | GNAQ | LPP | NCOA2 | PMS1 | SOCS1 |
| AFF3 | CDC73 | EPHB6 | GNAS | LRP1B | NCOA4 | PMS2 | SOX11 |
| AKAP9 | CDH1 | ERBB2 | GPR124 | LTF | NF1 | POT1 | SOX2 |
| AKT1 | CDH11 | ERBB3 | GRM8 | LTK | NF2 | POU5F1 | SRC |
| AKT2 | CDH2 | ERBB4 | GUCY1A2 | MAF | NFE2L2 | PPARG | SSX1 |
| AKT3 | CDH20 | ERCC1 | HCAR1 | MAFB | NFKB1 | PPP2R1A | STK11 |
| ALK | CDH5 | ERCC2 | HIF1A | MAGEA1 | NFKB2 | PRDM1 | STK36 |
| APC | CDK12 | ERCC3 | HLF | MAGI1 | NIN | PRKAR1A | SUFU |
| AR | CDK4 | ERCC4 | HNF1A | MALT1 | N KX2-1 | PRKDC | SYK |
| ARID1A | CDK6 | ERCC5 | HOOK3 | MAML2 | NLRP1 | PSIP1 | SYNE1 |
| ARID2 | CDK8 | ERG | HRAS | MAP2K1 | NOTCH 1 | PTCH1 | TAF1 |
| ARNT | CDKN2A | ESR1 | HSP90AA1 | MAP2K2 | NOTCH2 | PTEN | TAF1L |
| ASXL1 | CDKN2B | ETS1 | HSP90AB1 | MAP2K4 | NOTCH4 | PTGS2 | TAL1 |
| ATF1 | CDKN2C | ETV1 | ICK | MAP3K7 | NPM1 | PTPN11 | TBX22 |
| ATM | CEBPA | ETV4 | IDH1 | MAPK1 | NRAS | PTPRD | TCF12 |
| ATR | CHEK1 | EXT1 | IDH2 | MAPK8 | NSD1 | PTPRT | TCF3 |
| ATRX | CHEK2 | EXT2 | IGF1R | MARK1 | NTRK1 | RAD50 | TCF7L1 |
| AURKA | CIC | EZH2 | IGF2 | MARK4 | NTRK3 | RAF1 | TCF7L2 |
| AURKB | CKS1B | FAM123B | IGF2R | MBD1 | NUMA1 | RALGDS | TCL1A |
| AURKC | CMPK1 | FANCA | IKBKB | MCL1 | NUP214 | RARA | TET1 |
| AXL | COL1A1 | FANCC | IKBKE | MDM2 | NUP98 | RB1 | TET2 |
| BAI3 | CRBN | FANCD2 | IKZF1 | MDM4 | PAK3 | RECQL4 | TFE3 |
| BAP1 | CREB1 | FANCF | IL2 | MEN1 | PALB2 | REL | TGFBR2 |
| BCL10 | CREBBP | FANCG | IL21R | MET | PARP1 | RET | TGM7 |
| BCL11A | CRKL | FANCJ | IL6ST | MITF | PAX3 | RHOH | THBS1 |
| BCL11B | CRTC1 | FAS | IL7R | MLH1 | PAX5 | RNASEL | TIMP3 |
| BCL2 | CSF1R | FBXW7 | ING4 | MLL | PAX7 | RNF2 | TLR4 |
| BCL2L1 | CSMD3 | FGFR1 | IRF4 | M LL2 | PAX8 | RNF213 | TLX1 |
| BCL2L2 | CTNNA1 | FGFR2 | IRS2 | MLL3 | PBRM1 | ROS1 | TNFAIP3 |
| BCL3 | CTNNB1 | FGFR3 | ITGA10 | MLLT10 | PBX1 | RPS6KA2 | TNFRSF14 |
| BCL6 | CYLD | FGFR4 | ITGA9 | MMP2 | PDE4DIP | RRM1 | TNK2 |
| BCL9 | CYP2C19 | FH | ITGB2 | MN1 | PDGFB | RUNX1 | TOP1 |
| BCR | CYP2D6 | FLCN | ITGB3 | MPL | PDGFRA | RUNX1T1 | TP53 |
| BIRC2 | DAXX | FLI1 | JAK1 | MRE11A | PDGFRB | SAMD9 | TPR |
| BIRC3 | DCC | FLT1 | JAK2 | MSH2 | PER1 | SBDS | TRIM24 |
| BIRC5 | DDB2 | FLT3 | JAK3 | MSH6 | PGAP3 | SDHA | TRIM33 |
| BLM | DDIT3 | FLT4 | JUN | MTOR | PHOX2B | SDHB | TRIP11 |
| BLNK | DDR2 | FN1 | KAT6A | MTR | PIK3C2B | SDHC | TRRAP |
| BMPR1A | DEK | FOXL2 | KAT6B | MTRR | PIK3CA | SDHD | TSC1 |
| BRAF | DICER1 | FOXO1 | KDM5C | MUC1 | PIK3CB | Sep-09 | TSC2 |
| BRD3 | DNMT3A | FOXO3 | KDM6A | M UTYH | PIK3CD | SETD2 | TSHR |
| BTK | DPYD | FOXP1 | KDR | MYB | PIK3CG | SF3B1 | UBR5 |
| BUB1B | DST | FOXP4 | KEAP1 | MYC | PIK3R1 | SGK1 | UGT1A1 |
| CARD11 | EGFR | FZR1 | KIT | MYCL1 | PIK3R2 | SH2D1A | USP9X |
| CASC5 | EML4 | G6PD | KLF6 | MYCN | PIM1 | SMAD2 | VHL |
| CBL | EP300 | GATA1 | KRAS | MYD88 | PKHD1 | SMAD4 | WAS |
| | | | | | | | WHSC1 |
| | | | | | | | WRN |
| | | | | | | | WT1 |
| | | | | | | | XPA |
| | | | | | | | XPC |
| | | | | | | | XPO1 |
| | | | | | | | XRCC2 |
| | | | | | | | ZNF384 |
| | | | | | | | ZNF521 |

## Claims

1. A method for detecting or monitoring renal, bladder and/or prostate cancer, said method comprising analysing nucleic acid obtained from a urine sample from a subject, detecting the presence of an aberration selected from a single nucleotide variant, indel, deletion, amplication and/or fusion in a coding sequence, or increased or decreased gene expression from a coding sequence, of at least one biomarker from each of Tables 1 to 7, as well as,
the tumour mutational burden (TMB), and relating the presence of said biomarkers to the presence of malignancy wherein an algorithm indicative of the presence or level of malignancy is applied to the results obtained wherein
a score of '0' is applied to results which show no changes over wild type or normal expression profiles of the various biomarkers;
a score of '1' is applied in the case of the presence of an oncogenic mutation in a biomarker gene, or to the presence of 2 to 3 additional copies of a biomarker gene, or for the presence of an oncogenic gene fusion, or up to a 500 nRPM change in RNA expression of a biomarker gene, or for a TMB of <10mutations/Megabase;
a score of '2' is applied in the case of the presence of from 4 to 8 additional copies of a biomarker gene; or to 500 to 1500 nRPM change in RNA expression of a biomarker gene;
a score of '3' is applied in the case of the presence of from more than 8 additional copies of a biomarker gene; or to a >1500 nRPM change in RNA expression of a biomarker gene, or for a TMB of >10 mutations/Megabase;
wherein an overall score of 0 is indicative of no malignancy, a score of 1 to 2 is indicative of a malignancy with intermediate specificity and high sensitivity, a score of 3 to 5 is indicative of a malignancy with high specificity and high sensitivity, and a score in excess of 6 is indicative of malignancy with very high specificity and very high sensitivity.

2. A method according to claim 1 wherein the sample is a formalin fixed sample.

3. A method according to either claim 1 or 2 comprising, as a preliminary step, extracting nucleic acid from the sample.

4. A method according to any preceding claim wherein the results obtained are further used to identify susceptibility of a subject to PD-1 or PD-L1 targeted therapy or treatment.

5. A method according to any preceding claim wherein at least 10 of the biomarkers in Tables 1 to 7 are detected.

6. A method according to claim 5 wherein all the biomarkers listed in Tables 1 to 7 are detected.

7. A method according to any one of the preceding claims wherein the subject is an apparently healthy individual.

8. A method according to any one of claims 1 to 6, wherein the subject has been previously diagnosed with renal, bladder and/or prostate cancer and is undergoing treatment or therapy therefor, and wherein the method is carried out repeatedly over time, to monitor the efficacy of the treatment or therapy.

9. A method according to claim 8 wherein the results are used to decide to modify or expand on the said treatment or therapy.

10. A method according to any one of claims 1 to 6 wherein the subject is suffering from haematuria.

11. A method according to any one of the preceding claims which is carried out using a high-throughput assay platform.

12. A method according to any one of the preceding claims wherein the results are analysed to produce a customised recommendation for treatment of the cancer.

13. A system for for detecting or monitoring renal, bladder and/or prostate cancer, said system comprising:
a processor; and
a memory that stores code of an algorithm that, when executed by the processor, causes the computer system to:
receive input levels of the biomarkers of claim 1;
receive a further input level relating to the tumour mutational burden of nucleic acid in the urine sample;
analyze and transform the input levels via an algorithm to provide an output indicating the presence or level of malignancy present;
display the output on a graphical interface of the processor
wherein the algorithm applied is:
a score of '0' is applied to results which show no changes over wild type or normal expression profiles of the various biomarkers;
a score of '1' is applied in the case of the presence of an oncogenic mutation in a biomarker gene, or to the presence of 2 to 3 additional copies of a biomarker gene, or for the presence of an oncogenic gene fusion, or up to a 500 nRPM change in RNA expression of a biomarker gene, or for a TMB of <10mutations/Megabase;
a score of '2' is applied in the case of the presence of from 4 to 8 additional copies of a biomarker gene; or to 500 to 1500 nRPM change in RNA expression of a biomarker gene;
a score of '3' is applied in the case of the presence of from more than 8 additional copies of a biomarker gene; or >1500 nRPM change in RNA expression of a biomarker gene, or for a TMB of >10 mutations/Megabase;
wherein an overall score of 0 is indicative of no malignancy, a score of 1 to 2 is indicative of a malignancy with intermediate specificity and high sensitivity, a score of 3 to 5 is indicative of a malignancy with high specificity and high sensitivity, and a score in excess of 6 is indicative of malignancy with very high specificity and very high sensitivity.

14. A system according to claim 13 wherein the memory further comprises code to provide a customized recommendation for the treatment of the subject, based upon the input levels.

15. A system according to claim 14 wherein the customized recommendation is displayed on a graphical interface of the processor.

16. A non-transitory computer-readable medium storing instructions that, when executed by a processor, cause a computer system to detect or monitor the level of renal, bladder nd/or prostate cancer in a urine sample of a subject, by:
receiving input levels of the biomarkers of claim 1 and
receiving a further input level relating to the tumour mutational burden of nucleic acid in the urine sample;
analyzing and transforming the input levels via an algorithm to provide an output indicating the presence or level of malignancy present;
displaying the output on a graphical interface of the processor
wherein the algorithm applied is:
a score of '0' is applied to results which show no changes over wild type or normal expression profiles of the various biomarkers;
a score of '1' is applied in the case of the presence of an oncogenic mutation in a biomarker gene, or to the presence of 2 to 3 additional copies of a biomarker gene, or for the presence of an oncogenic gene fusion, or up to a 500 nRPM change in RNA expression of a biomarker gene, or for a TMB of <10mutations/Megabase;
a score of '2' is applied in the case of the presence of from 4 to 8 additional copies of a biomarker gene; or to 500 to 1500 nRPM change in RNA expression of a biomarker gene;
a score of '3' is applied in the case of the presence of from more than 8 additional copies of a biomarker gene; or >1500 nRPM change in RNA expression of a biomarker gene, or for a TMB of >10 mutations/Megabase;
wherein an overall score of 0 is indicative of no malignancy, a score of 1 to 2 is indicative of a malignancy with intermediate specificity and high sensitivity, a score of 3 to 5 is indicative of a malignancy with high specificity and high sensitivity, and a score in excess of 6 is indicative of malignancy with very high specificity and very high sensitivity.

17. A non-transitory computer-readable medium according to claim 16 further storing instructions for developing a customized recommendation for treatment of the subject based upon the input levels and displaying the customized recommendation on a graphical interface of the processor.

## Patentansprüche

1. Verfahren zum Erkennen oder Überwachen von Nieren-, Blasen- und/oder Prostatakrebs, dieses Verfahren umfasst das Analysieren von Nukleinsäure aus einer Urinprobe einer Person, das Erkennen des Vorhandenseins einer Anomalie ausgewählt aus einer Einzelnukleotidvariante, Indel, Deletion, Verstärkung und/oder Fusion in einer codierenden Sequenz oder erhöhte oder verminderte Genexpression aus einer codierenden Sequenz, von mindestens einem Biomarker aus einer der Tabellen 1 bis 7, ebenso,
der Belastung durch Tumormutationen (TMB), und Beziehen des Vorhandenseins dieser Biomarker auf das Vorhandensein von Malignität, wobei ein Algorithmus, der indikativ für das Vorhandensein oder den Grad der Malignität ist, auf die erlangten Resultate angewendet wird, wobei
ein Score von '0' auf Resultate angewendet wird, die keine Änderungen zum Wildtyp oder zu normalen Expressionsprofilen der verschiedenen Biomarker zeigen;
ein Score von '1' für den Fall des Vorhandenseins einer onkogenischen Mutation in einem Biomarker-Gen, oder des Vorhandenseins von 2 bis 3 zusätzlichen Kopien eines Biomarker-Gens, oder für das Vorhandensein einer onkogenischen Genfusion oder über 500 nRPM-Änderung in der RNA-Expression eines Biomarker-Gens, oder für eine TMB von <10 Mutationen/Megabase angewendet wird;
ein Score von ,2' für den Fall des Vorhandenseins von 4 bis 8 zusätzlichen Kopien eines Biomarker-Gens, oder einer Änderung von 500 bis 1500 nRPM in der RNA-Expression eines Biomarker-Gens angewendet wird;
ein Score von ,3' für den Fall des Vorhandenseins von mehr als 8 zusätzlichen Kopien eines Biomarker-Gens; oder auf eine Änderung von >1500 nRPM in der RNA-Expression eines Biomarker-Gens, oder für eine TMB von >10 Mutationen/Megabase angewendet wird;
wobei ein Gesamtscore von 0 indikativ für eine nicht vorhandene Malignität, ein Score von 1 bis 2 indikativ für eine Malignität mit mittlerer Spezifität und hoher Sensitivität, ein Score von 3 bis 5 indikativ für eine Malignität mit hoher Spezifität und hoher Sensitivität und ein Score über 6 indikativ für eine Malignität mit sehr hoher Spezifität und sehr hoher Sensitivität ist.

2. Verfahren nach Anspruch 1, wobei die Probe eine formalin-fixierte Probe ist.

3. Verfahren nach Anspruch 1 oder 2, umfassend, als ein vorbereitender Schritt, das Extrahieren von Nukleinsäure aus der Probe.

4. Verfahren nach einem der vorhergehenden Ansprüche, wobei die erlangten Resultate ferner verwendet werden, um die Empfänglichkeit einer Person für eine auf PD-1- oder PD-L1 zielende Therapie oder -Behandlung zu identifizieren.

5. Verfahren nach einem der vorhergehenden Ansprüche, wobei mindestens 10 der Biomarker in den Tabellen 1 bis 7 erkannt werden.

6. Verfahren nach Anspruch 5, wobei alle in den Tabellen 1 bis 7 aufgelisteten Biomarker erkannt werden.

7. Verfahren nach einem der vorhergehenden Ansprüche, wobei die Person ein scheinbar gesunder Mensch ist.

8. Verfahren nach einem der Ansprüche 1 bis 6, wobei die Person zuvor die Diagnose eines Nieren-, Blasen- und/oder Prostatakrebses erhalten hat und sich einer diesbezüglichen Behandlung oder Therapie unterziehen musste, und wobei das Verfahren über die Zeit wiederholt ausgeführt wird, um die Wirksamkeit der Behandlung oder Therapie zu überwachen.

9. Verfahren nach Anspruch 8, wobei die Resultate verwendet werden, um zu entscheiden, ob diese Behandlung oder Therapie geändert oder erweitert werden soll.

10. Verfahren nach einem der Ansprüche 1 bis 6, wobei die Person unter Hämaturie leidet.

11. Verfahren nach einem der vorhergehenden Ansprüche, das unter Verwendung einer Hochdurchsatz-Assay-Plattform ausgeführt wird.

12. Verfahren nach einem der vorhergehenden Ansprüche, wobei die Resultate analysiert werden, um eine maßgeschneiderte Empfehlung für die Behandlung des Krebses zu erstellen.

13. System für das Erkennen oder Überwachen von Nieren-, Blasen- und/oder Prostatakrebs, dieses System umfassend:
einen Prozessor; und
einen Speicher, der den Code eines Algorithmus speichert, der, wenn er von dem Prozessor ausgeführt wird, das Computersystem zu Folgendem veranlasst:
Empfangen von Eingangslevel der Biomarker von Anspruch 1
Empfangen eines weiteren Eingangslevels in Bezug auf die Belastung durch Tumormutationen der Nukleinsäure in der Urinprobe;
Analysieren und Umwandeln der Eingangslevel über einen Algorithmus, um ein Ergebnis bereitzustellen, das das Vorhandensein oder den gegebenen Malignitätsgrad anzeigt;
Anzeigen des Ergebnisses auf einer grafischen Benutzeroberfläche des Prozessors
wobei sich der angewendete Algorithmus wie folgt darstellt:
Ein Score von '0' wird auf Resultate angewendet, die keine Änderungen zum Wildtyp oder zu normalen Expressionsprofilen der verschiedenen Biomarker zeigen;
ein Score von '1' wird für den Fall des Vorhandenseins einer onkogenischen Mutation in einem Biomarker-Gen, oder des Vorhandenseins von 2 bis 3 zusätzlichen Kopien eines Biomarker-Gens, oder für das Vorhandensein einer onkogenischen Genfusion oder über 500 nRPM-Änderung in der RNA-Expression eines Biomarker-Gens, oder für eine TMB von <10 Mutationen/Megabase angewendet;
ein Score von ,2' wird für den Fall des Vorhandenseins von 4 bis 8 zusätzlichen Kopien eines Biomarker-Gens, oder einer Änderung von 500 bis 1500 nRPM in der RNA-Expression eines Biomarker-Gens angewendet;
ein Score von ,3' wird für den Fall des Vorhandenseins von mehr als 8 zusätzlichen Kopien eines Biomarker-Gens; oder auf eine Änderung >1500 nRPM in der RNA-Expression eines Biomarker-Gens, oder für eine TMB von >10 Mutationen/Megabase angewendet;
wobei ein Gesamtscore von 0 indikativ für eine nicht vorhandene Malignität, ein Score von 1 bis 2 indikativ für eine Malignität mit mittlerer Spezifität und hoher Sensitivität, ein Score von 3 bis 5 indikativ für eine Malignität mit hoher Spezifität und hoher Sensitivität und ein Score über 6 indikativ für eine Malignität mit sehr hoher Spezifität und sehr hoher Sensitivität ist.

14. System nach Anspruch 13, wobei der Speicher ferner einen Code umfasst, um eine maßgeschneiderte Empfehlung für die Behandlung der Person, basierend auf den Eingangslevels, zu erstellen.

15. System nach Anspruch 14, wobei die maßgeschneiderte Empfehlung auf einer grafischen Benutzeroberfläche des Prozessors angezeigt wird.

16. Ein nichttransitorisches computerlesbares Medium, das Anweisungen speichert, die, wenn sie von einem Prozessor ausgeführt werden, ein Computersystem dazu veranlassen, den Grad des Nieren-, Blasen- und/oder Prostatakrebses in einer Urinprobe einer Person zu erkennen oder zu überwachen, durch:
Empfangen von Eingangslevel der Biomarker von Anspruch 1 und
Empfangen eines weiteren Eingangslevels in Bezug auf die Belastung durch Tumormutationen der Nukleinsäure in der Urinprobe;
Analysieren und Umwandeln der Eingangslevel über einen Algorithmus, um ein Ergebnis bereitzustellen, das das Vorhandensein oder den gegebenen Malignitätsgrad anzeigt;
Anzeigen des Ergebnisses auf einer grafischen Benutzeroberfläche des Prozessors
wobei sich der angewendete Algorithmus wie folgt darstellt:
ein Score von '0' wird auf Resultate angewendet, die keine Änderungen zum Wildtyp oder zu normalen Expressionsprofilen der verschiedenen Biomarker zeigen;
ein Score von '1' wird für den Fall des Vorhandenseins einer onkogenischen Mutation in einem Biomarker-Gen, oder des Vorhandenseins von 2 bis 3 zusätzlichen Kopien eines Biomarker-Gens, oder für das Vorhandensein einer onkogenischen Genfusion oder über A 500 nRPM-Änderung in der RNA-Expression eines Biomarker-Gens, oder für eine TMB von <10 Mutationen/Megabase angewendet;
ein Score von ,2' wird für den Fall des Vorhandenseins von 4 bis 8 zusätzlichen Kopien eines Biomarker-Gens, oder einer Änderung von 500 bis 1500 nRPM in der RNA-Expression eines Biomarker-Gens angewendet;
ein Score von ,3' wird für den Fall des Vorhandenseins von mehr als 8 zusätzlichen Kopien eines Biomarker-Gens; oder auf eine Änderung >1500 nRPM in der RNA-Expression eines Biomarker-Gens, oder für eine TMB von >10 Mutationen/Megabase angewendet;
wobei ein Gesamtscore von 0 indikativ für eine nicht vorhandene Malignität, ein Score von 1 bis 2 indikativ für eine Malignität mit mittlerer Spezifität und hoher Sensitivität, ein Score von 3 bis 5 indikativ für eine Malignität mit hoher Spezifität und hoher Sensitivität und ein Score über 6 indikativ für eine Malignität mit sehr hoher Spezifität und sehr hoher Sensitivität ist.

17. Ein nichttransitorisches computerlesbares Medium nach Anspruch 16, das ferner Anweisungen für das Entwickeln einer maßgeschneiderten Empfehlung für die Behandlung einer Person auf der Grundlage der Eingangslevel speichert und die maßgeschneiderte Empfehlung auf einer grafischen Benutzeroberfläche des Prozessors anzeigt.

## Revendications

1. Méthode de détection ou de surveillance du cancer du rein, de la vessie et/ou de la prostate, ladite méthode comprenant l'analyse de l'acide nucléique obtenu à partir d'un échantillon d'urine en provenance d'un sujet, la détection de la présence d'une aberration choisie parmi un variant nucléotidique simple, un indel, une délétion, une amplication et/ou une fusion dans une séquence codante, ou une augmentation ou une diminution de l'expression génique à partir d'une séquence codante, d'au moins un biomarqueur de chacun des tableaux 1 à 7, ainsi que,
la charge mutationnelle tumorale (TMB), et la mise en relation de la présence desdits biomarqueurs avec la présence d'une malignité, un algorithme indiquant la présence ou le niveau de malignité étant appliqué aux résultats obtenus, dans laquelle
un score de < 0 > est appliqué aux résultats qui ne montrent aucun changement par rapport au type sauvage ou aux profils d'expression normaux des différents biomarqueurs ;
un score de < 1 > est appliqué en cas de présence d'une mutation oncogène dans un gène biomarqueur, ou en cas de présence de 2 à 3 copies supplémentaires d'un gène biomarqueur, ou en cas de présence d'une fusion de gènes oncogènes, ou de changement jusqu'à 500 nRPM (lectures normalisées par million) dans l'expression de l'ARN d'un gène biomarqueur, ou en cas de TMB <10 mutations/mégabase ;
un score de < 2 > est appliqué en cas de présence de 4 à 8 copies supplémentaires d'un gène biomarqueur ; ou d'un changement de 500 à 1500 nRPM dans l'expression de l'ARN d'un gène biomarqueur ;
un score de < 3 > est appliqué en cas de présence à partir de plus de 8 copies supplémentaires d'un gène biomarqueur, ou en cas de changement jusqu'à 1500 nRPM dans l'expression de l'ARN d'un gène biomarqueur, ou en cas de TMB >10 mutations/mégabase ;
dans laquelle un score global de 0 indique l'absence de malignité, un score de 1 à 2 indique une malignité avec une spécificité intermédiaire et une sensibilité élevée, un score de 3 à 5 indique une malignité avec une spécificité élevée et une sensibilité élevée, et un score supérieur à 6 indique une malignité avec une spécificité très élevée et une sensibilité très élevée.

2. Méthode selon la revendication 1, dans laquelle l'échantillon est un échantillon fixé à la formaline.

3. Méthode selon la revendication 1 ou 2 comprenant, comme étape préliminaire, l'extraction de l'acide nucléique de l'échantillon.

4. Méthode selon une quelconque revendication précédente, dans laquelle les résultats obtenus sont en outre utilisés pour identifier la sensibilité d'un sujet à une thérapie ou à un traitement ciblant PD-1 ou PD-L1.

5. Méthode selon une quelconque revendication précédente, dans laquelle au moins 10 parmi les biomarqueurs des tableaux 1 à 7 sont détectés

6. Méthode selon la revendication 5, dans laquelle tous les biomarqueurs énumérés dans les tableaux 1 à 7 sont détectés.

7. Méthode selon l'une quelconque des revendications précédentes, dans laquelle le sujet est une personne apparemment en bonne santé.

8. Méthode selon l'une quelconque des revendications 1 à 6, dans laquelle le sujet a déjà été préalablement diagnostiqué avec un cancer du rein, de la vessie et/ou de la prostate et suit un traitement ou une thérapie à cet effet et dans laquelle la méthode est effectuée de manière répétée dans le temps, afin de surveiller l'efficacité du traitement ou de la thérapie.

9. Méthode selon la revendication 8, dans laquelle les résultats sont utilisés pour décider de modifier ou d'étendre ledit traitement ou ladite thérapie.

10. Méthode selon l'une quelconque des revendications 1 à 6 dans laquelle le sujet souffre d'hématurie.

11. Méthode selon l'une quelconque des revendications précédentes, qui est réalisée à l'aide d'une plateforme de dosage à haut débit.

12. Méthode selon l'une quelconque des revendications précédentes, dans laquelle les résultats sont analysés pour produire une recommandation personnalisée pour le traitement du cancer.

13. Système de détection ou de surveillance du cancer du rein, de la vessie et/ou de la prostate, ledit système comprenant :
un processeur ; et
une mémoire qui mémorise le code d'un algorithme qui, lorsqu'il est exécuté par le processeur, amène le système informatique à :
recevoir les niveaux d'entrée des biomarqueurs de la revendication 1
recevoir un niveau d'entrée supplémentaire se rapportant à la charge mutationnelle tumorale de l'acide nucléique dans l'échantillon d'urine ;
analyser et transformer les niveaux d'entrée par l'intermédiaire d'un algorithme afin de fournir une sortie indiquant la présence ou le niveau de malignité ;
afficher la sortie sur une interface graphique du processeur
dans lequel l'algorithme appliqué est :
un score de < 0 > est appliqué aux résultats qui ne montrent aucun changement par rapport au type sauvage ou aux profils d'expression normaux des différents biomarqueurs ;
un score de < 1 > est appliqué en cas de présence d'une mutation oncogène dans un gène biomarqueur, ou en cas de présence de 2 à 3 copies supplémentaires d'un gène biomarqueur, ou en cas de présence d'une fusion de gènes oncogènes, ou de changement jusqu'à 500 nRPM (lectures normalisées par million) dans l'expression de l'ARN d'un gène biomarqueur, ou en cas de TMB <10 mutations/mégabase ;
un score de < 2 > est appliqué en cas de présence de 4 à 8 copies supplémentaires d'un gène biomarqueur ; ou d'un changement de 500 à 1500 nRPM dans l'expression de l'ARN d'un gène biomarqueur ;
un score de < 3 > est appliqué en cas de présence à partir de plus de 8 copies supplémentaires d'un gène biomarqueur ; ou de changement >1500 nRPM dans l'expression de l'ARN d'un gène biomarqueur, ou pour un TMB >10 mutations/mégabase ;
dans laquelle un score global de 0 indique l'absence de malignité, un score de 1 à 2 indique une malignité avec une spécificité intermédiaire et une sensibilité élevée, un score de 3 à 5 indique une malignité avec une spécificité élevée et une sensibilité élevée, et un score supérieur à 6 indique une malignité avec une spécificité très élevée et une sensibilité très élevée.

14. Système selon la revendication 13, dans lequel la mémoire comprend en outre un code permettant de fournir une recommandation personnalisée destinée au traitement du sujet, sur la base des niveaux d'entrée.

15. Système selon la revendication 14, dans lequel la recommandation personnalisée est affichée sur une interface graphique du processeur.

16. Support non transitoire lisible par ordinateur mémorisant des instructions qui, lorsqu'elles sont exécutées par un processeur, amènent un système informatique à détecter ou à surveiller le niveau de cancer du rein, de la vessie et/ou de la prostate dans un échantillon d'urine d'un sujet, en :
recevant les niveaux d'entrée des biomarqueurs de la revendication 1 et
recevant un niveau d'entrée supplémentaire se rapportant à la charge mutationnelle tumorale de l'acide nucléique dans l'échantillon d'urine ;
analysant et transformant les niveaux d'entrée par l'intermédiaire d'un algorithme afin de fournir une sortie indiquant la présence ou le niveau de malignité ;
affichant la sortie sur une interface graphique du processeur
dans lequel l'algorithme appliqué est :
un score de < 0 > est appliqué aux résultats qui ne montrent aucun changement par rapport au type sauvage ou aux profils d'expression normaux des différents biomarqueurs ;
un score de < 1 > est appliqué en cas de présence d'une mutation oncogène dans un gène biomarqueur, ou en cas de présence de 2 à 3 copies supplémentaires d'un gène biomarqueur, ou en cas de présence d'une fusion de gènes oncogènes, ou de changement jusqu'à 500 nRPM dans l'expression de l'ARN d'un gène biomarqueur, ou en cas de TMB <10 mutations/mégabase ;
un score de < 2 > est appliqué en cas de présence de 4 à 8 copies supplémentaires d'un gène biomarqueur ; ou d'un changement de 500 à 1500 nRPM dans l'expression de l'ARN d'un gène biomarqueur ;
un score de < 3 > est appliqué en cas de présence à partir de plus de 8 copies supplémentaires d'un gène biomarqueur ; ou de changement >1500 nRPM dans l'expression de l'ARN d'un gène biomarqueur, ou pour un TMB >10 mutations/mégabase ;
dans laquelle un score global de 0 indique l'absence de malignité, un score de 1 à 2 indique une malignité avec une spécificité intermédiaire et une sensibilité élevée, un score de 3 à 5 indique une malignité avec une spécificité élevée et une sensibilité élevée, et un score supérieur à 6 indique une malignité avec une spécificité très élevée et une sensibilité très élevée.

17. Support non transitoire lisible par ordinateur selon la revendication 16, mémorisant en outre des instructions destinées à développer une recommandation personnalisée servant au traitement du sujet sur la base des niveaux d'entrée et affichant la recommandation personnalisée sur une interface graphique du processeur.
